Europäisches Patentamt

European Patent Office

Office européen des brevets

(11) Publication number: **0 547 026 A1**

## (12) EUROPEAN PATENT APPLICATION

(21) Application number: **93100662.1**

(22) Date of filing: **17.09.87**

(51) Int. Cl.5: **C12N 15/27**, C12P 21/02, C07K 13/00, C12N 1/21

This application was filed on 18 - 01 - 1993 as a divisional application to the application mentioned under INID code 60.

(30) Priority: **17.09.86 JP 220750/86**
**19.01.87 JP 11025/87**
**02.07.87 JP 166388/87**

(43) Date of publication of application:
**16.06.93 Bulletin 93/24**

(60) Publication number of the earlier application in accordance with Art.76 EPC: **0 261 592**

(84) Designated Contracting States:
**CH DE ES FR GB IT LI NL SE**

(71) Applicant: **OTSUKA PHARMACEUTICAL CO., LTD.**
**9, Kandatsukasa-cho 2-chome**
**Chiyoda-ku Tokyo-to(JP)**

(72) Inventor: **Takahashi, Masayuki**
**79-7, Aza-Nakanokoshi, Kizuno**
**Ootsu-cho, Naruto-shi, Tokushima-ken(JP)**
Inventor: **Hirato, Tohru**
**463-10, Kagasuno, Kawauchi-cho**
**Tokushima-shi, Tokushima-ken(JP)**
Inventor: **Nakai, Satoru**
**67-4, Aza-Minamikawamuko, Hiroshima**
**Matsushige-cho, Itano-gun,**
**Tokushima-ken(JP)**
Inventor: **Hong, Yeong-Man, Sanraunjihausu 105**
**129-4, Aza-Miyanonishi, Kitahama**
**Muya-cho, Naruto-shi, Tokushima-ken(JP)**
Inventor: **Kouno, Naomi,**
**Hachibancho-River-Haitsu 404**
**8-17, Sakohachiban-cho**
**Tokushima-shi, Tokushima-ken(JP)**
Inventor: **Hirai, Yoshikatsu**
**5-49, Aza-Ekogawa, Shinkirai**
**Kitajima-cho, Itano-gun, Tokushima-ken(JP)**

(74) Representative: **Kolb, Helga, Dr. Dipl.-Chem. et al**
**Hoffmann, Eitle & Partner Patentanwälte**
**Arabellastrasse 4**
**W-8000 München 81 (DE)**

(54) Gene coding for human colony-stimulating factors.

(57) A gene which codes for a biologically active human M-CSF and has a nucleotide sequence coding for the amino acid sequence of the formula (1) or a portion thereof; an expression vector including the gene defined above and capable of expressing a biologically active human M-CSF; and a transformant habouring the expression vector defined above and capable of producing a biologically active human M-CSF.

The present invention relates to a novel gene coding for human colony-stimulating factors (CSFs) useful as drugs.

Generally, specific proliferation and differentiation factors are required for the proliferation and differentiation of hematopoietic cells. Various such factors participate in the differentiation and proliferation of hematopoietic cells into various blood cells, such as erythrocytes, granulocytes, macrophages, eosinophils, platelets and lymphocytes (Yasusada Miura, "Blood Stem Cells," Chugai Igaku Sha, 1983). Of these factors, CSFs are known as factors which stimulate the proliferation and differentiation of granulocyte precursor cells and macrophage precursor cells. Known as such CSFs are G-CSF which is specific to the formation of granulocytes, M-CSF which is specific to the formation of macrophages, and GM-CSF which acts to form both granulocytes and macrophages, and further multi-CSF(IL-3) is known which stimulates pluripotent stem cells.

The above-mentioned CSFs, because of their biological activity, are thought to alleviate the leukopenia resulting from cancer chemotherapy and radiotherapy as a common drawback of these therapies. Clininal research on CSFs is conducted from this viewpoint.

The CSFs are also known to have activity to promote the function of leukocytes (Lopez, A.F. et al., J. Immunol., 131, 2983 (1983), Handam, E. et al., same, 122, 1134 (1979) and Vadas, M.A. et al., same, 130, 795 (1983)) and are therefore found effective as drugs for preventing and curing various infectious diseases.

Furthermore, the CSFs are found effective for curing myelogenous leukemia because of its differentiation inducing activity (Metcalf, D. et al., Int. J. Cancer, 30, 773 (1982)).

The activity of CSFs is found, for example, in the cultures of fetal cells, spleen cells, etc. in human urine and in the culture media of various incubated cell lines, and active fractions thereof are separated off and used. However, the CSF of any origin contains large quantities of analogous extraneous substances or the like derived from the starting material and is in itsefl low in concentration, so that these problems must be solved in preparing the CSF. In respect of homogeneity, yield, ease of operation, etc., therefore, methods still remain to be developed of commercially preparing CSFs as drugs.

We have already established "AGR-ON," a cell line derived from human leukemic T cells, which is capable of producing a large quantity of CSF at all times in a homogeneous state, and filed for patent application an invention relating to this cell line (Unexamined Japanese Patent Publication SHO 59-169489). We have also developed a process for preparing a CSF from AGR-ON easily in a pure form in a high yield by repeating experiments on the purification of the CSF produced by AGR-ON, further clarified the structural and biochemical characteristics of the resulting CSF, and filed for patent application an invention relating to the CSF as a substance with such features (Unexamined Japanese Patent Publication SHO 61-12850).

The CSF disclosed by the above invention is M-CSF, i.e., a glycoprotein which acts on normal bone marrow cells to promote the differentiation and proliferation of macrophages and which is characterized by the following physicochemical properties. The CSF is termed "AGR-ON•CSF."

a) Molecular weight

33000 to 43000 daltons as determined by SDS polyacrylamide gel electrophoresis under a non-reducing condition, and 23000 to 40000 daltons as determined by gel filtration under a non-reducing condition in the presence of SDS.

b) N-terminal amino acid sequence of the protein portion

The sequence of the following primary structural formula.

```
Val-Ser-Glu-Tyr-Cys-Ser-His-Met-Ile-Gly-Ser-Gly-His-

Leu-Gln-Ser-Leu-Gln-Arg-Leu-Ile-Asp-Ser-Gln-Met-Glu-

Thr
```

The main object of the present invention is to provide techniques for preparing and supplying the human M-CSF in large quantities with ease by genetic engineering techniques, and a gene useful for the techniques.

We have conducted research subsequent to the foregoing research efforts and accomplished the present invention by extracting messenger RNAs (mRNAs) from AGR-ON from which AGR-ON•CSF is derived, preparing DNAs (cDNAs) complementary to the mRNAs therefrom, selecting a cDNA coding for the desired M-CSF based on the amino acid sequence information of AGR-ON•CSF, preparing a vector containing the cDNA, introducing the vector into a host cell to obtain a transformant and incubating the transformant to obtain a culture in which M-CSF having the desired biological activity is produced.

More specifically, the present invention provides a gene which is characterized in that the gene codes for a biologically active human M-CSF molecule based on the information of an amino acid sequence of the following formula (1).

```
      1                                10
      Met-Thr-Ala-Pro-Gly-Ala-Ala-Gly-Arg-Cys-
                                       20
      Pro-Pro-Thr-Thr-Trp-Leu-Gly-Ser-Leu-Leu-
                                       30
      Leu-Leu-Val-Cys-Leu-Leu-Ala-Ser-Arg-Ser-
                                       40
      Ile-Thr-Glu-Glu-Val-Ser-Glu-Tyr-Cys-Ser-
                                       50
      His-Met-Ile-Gly-Ser-Gly-His-Leu-Gln-Ser-
                                       60
      Leu-Gln-Arg-Leu-Ile-Asp-Ser-Gln-Met-Glu-
                                       70
      Thr-Ser-Cys-Gln-Ile-Thr-Phe-Glu-Phe-Val-
                                       80
      Asp-Gln-Glu-Gln-Leu-Lys-Asp-Pro-Val-Cys-
                                       90
      Tyr-Leu-Lys-Lys-Ala-Phe-Leu-Leu-Val-Gln-
                                      100
       X -Ile-Met-Glu-Asp-Thr-Met-Arg-Phe-Arg-
                                      110
      Asp-Asn-Thr-Pro-Asn-Ala-Ile-Ala-Ile-Val-
                                      120
      Gln-Leu-Gln-Glu-Leu-Ser-Leu-Arg-Leu-Lys-
                                      130
      Ser-Cys-Phe-Thr-Lys-Asp-Tyr-Glu-Glu-His-
                                      140
      Asp-Lys-Ala-Cys-Val-Arg-Thr-Phe-Tyr-Glu-
                                      150
      Thr-Pro-Leu-Gln-Leu-Leu-Glu-Lys-Val-Lys-
                                      160
      Asn-Val-Phe-Asn-Glu-Thr-Lys-Asn-Leu-Leu-
                                      170
      Asp-Lys-Asp-Trp-Asn-Ile-Phe-Ser-Lys-Asn-
                                      180
      Cys-Asn-Asn-Ser-Phe-Ala-Glu-Cys-Ser-Ser-
                                      190
      Gln-Asp-Val-Val-Thr-Lys-Pro-Asp-Cys-Asn-
                                      200
      Cys-Leu-Tyr-Pro-Lys-Ala-Ile-Pro-Ser-Ser-
                                      210
      Asp-Pro-Ala-Ser-Val-Ser-Pro-His-Gln-Pro-
                                      220
      Leu-Ala-Pro-Ser-Met-Ala-Pro-Val-Ala-Gly-
                                      230
      Leu-Thr-Trp-Glu-Asp-Ser-Glu-Gly-Thr-Glu-
                                      240
      Gly-Ser-Ser-Leu-Leu-Pro-Gly-Glu-Gln-Pro-
                                      250
      Leu-His-Thr-Val-Asp-Pro-Gly-Ser-Ala-Lys-
```

```
                                        260
Gln-Arg-Pro-Pro-Arg-Ser-Thr-Cys-Gln-Ser-
                                        270
Phe-Glu-Pro-Pro-Glu-Thr-Pro-Val-Val-Lys-
                                        280
Asp-Ser-Thr-Ile-Gly-Gly-Ser-Pro-Gln-Pro-
                                        290
Arg-Pro-Ser-Val-Gly-Ala-Phe-Asn-Pro-Gly-
                                        300
Met-Glu-Asp-Ile-Leu-Asp-Ser-Ala-Met-Gly-
                                        310
Thr-Asn-Trp-Val-Pro-Glu-Glu-Ala-Ser-Gly-
                                        320
Glu-Ala-Ser-Glu-Ile-Pro-Val-Pro-Gln-Gly-
                                        330
Thr-Glu-Leu-Ser-Pro-Ser-Arg-Pro-Gly-Gly-
                                        340
Gly-Ser-Met-Gln-Thr-Glu-Pro-Ala-Arg-Pro-
                                        350
Ser-Asn-Phe-Leu-Ser-Ala-Ser-Ser-Pro-Leu-
                                        360
Pro-Ala-Ser-Ala-Lys-Gly-Gln-Gln-Pro-Ala-
                                        370
Asp-Val-Thr-Gly-Thr-Ala-Leu-Pro-Arg-Val-
                                        380
Gly-Pro-Val-Arg-Pro-Thr-Gly-Gln-Asp-Trp-
                                        390
Asn-His-Thr-Pro-Gln-Lys-Thr-Asp-His-Pro-
                                        400
Ser-Ala-Leu-Leu-Arg-Asp-Pro-Pro-Glu-Pro-
                                        410
Gly-Ser-Pro-Arg-Ile-Ser-Ser-Pro-Arg-Pro-
                                        420
Gln-Gly-Leu-Ser-Asn-Pro-Ser-Thr-Leu-Ser-
                                        430
Ala-Gln-Pro-Gln-Leu-Ser-Arg-Ser-His-Ser-
                                        440
Ser-Gly-Ser-Val-Leu-Pro-Leu-Gly-Glu-Leu-
                                        450
Glu-Gly-Arg-Arg-Ser-Thr-Arg-Asp-Arg-Arg-
                                        460
Ser-Pro-Ala-Glu-Pro-Glu-Gly-Gly-Pro-Ala-
                                        470
Ser-Glu-Gly-Ala-Ala-Arg-Pro-Leu-Pro-Arg-
                                        480
Phe-Asn-Ser-Val-Pro-Leu-Thr-Asp-Thr-Gly-
                                        490
His-Glu-Arg-Gln-Ser-Glu-Gly-Ser-Ser-Ser-
                                        500
Pro-Gln-Leu-Gln-Glu-Ser-Val-Phe-His-Leu-
```

```
                                        510
        Leu-Val-Pro-Ser-Val-Ile-Leu-Val-Leu-Leu-
                                        520
        Ala-Val-Gly-Gly-Leu-Leu-Phe-Tyr-Arg-Trp-
                                        530
        Arg-Arg-Arg-Ser-His-Gln-Glu-Pro-Gln-Arg-
                                        540
        Ala-Asp-Ser-Pro-Leu-Glu-Gln-Pro-Glu-Gly-
                                        550
        Ser-Pro-Leu-Thr-Gln-Asp-Asp-Arg-Gln-Val-
                  554
        Glu-Leu-Pro-Val
```

wherein X is Tyr or Asp.

Peptides and amino acids are herein referred to by symbols according to the amino acid nomenclature adopted by IUPAC or by symbols conventionally used in the art. Nucleotide sequences and nucleic acids are also expressed similarly.

Using the gene of the invention, human M-CSF can be produced easily in large quantities by a genetic engineering process. As already described, the M-CSF thus obtained, because of its biological activity, is useful especially in the field of medicinals as a drug for preventing and curing diseases involving leukopenia, as an auxiliary agent for the bone marrow transplantation, as a drug for preventing and curing various infectious diseases, as an anticancer drug, etc.

The gene of the invention will be described below in detail.

The gene of the invention is prepared, for example, from human cells having ability to produce M-CSF, more specifically and advantageously from mRNA separated from AGR-ON. The preparation of the gene will hereinafter be described with reference to AGR-ON although the gene can be similarly prepared from other cells. AGR-ON is a human cell line derived from human leukemic T cells and having the characteristics described in Unexamined Japanese Patent Publication SHO 59-169489. The cell line is deposited in American Type Culture Collection (ATCC) as ATCC Deposition No.CRL-8199.

The mRNA is isolated from AGR-ON basically by a usual extraction procedure. Stated more specifically, AGR-ON is cultured, for example, in CEM medium, CMRL-1066 medium, DM-160 medium, Eagle's minimum essential medium (Eagle's MEM), Fisher's medium, F-10 medium, F-12 medium, L-15 medium, NCTC-109 medium, RPMI-1640 medium or the like, with fetal calf serum (FCS) or like serum, or albumin or like serum component added thereto when desired. AGR-ON is inoculated in the medium at a concentration of about $1 \times 10^4$ to $1 \times 10^7$ cells/ml and is incubated by a usual method, such as culturing in $CO_2$ incubator, at about 30 to about 40°C, preferably at about 37°C, for 1 to 5 days. When the CSF has been produced and accumulated, the cultured cells are lysed partially or completely with a suitable surfactant, such as SDS, NP-40, Triton X100 or deoxycholic acid, or by a homogenizer or by some physical method such as freeze-thaw method. The chromosome DNA is then sheared to some extent by POLYTORON (product of Kinematica, Switzerland) or like mixer or a syringe. Proteins are thereafter removed from a nucleic acid fraction for the extraction of total RNA. Generally used for this procedure is, for example, the CsCl ultracentrifugation method or phenol-chloroform extraction (Chirgwin, J.M. et al., Biochemistry, 18, 5294 (1979)).

To prevent the degradation of RNA with RNase, the above method or procedure can be carried out in the presence of RNase inhibitors, such as heparin, polyvinyl sulfate, diethyl pyrocarbonate, vanadyl-ribonucleoside complex, bentonite or macaloid.

mRNAs can be separated from the extracted RNA and purified by a column method or batchwise method using, for example, oligo dT-cellulose (product of Collaborative Research Inc.), poly-U-Sepharose (product of Pharmacia Fine Chemicals), Sepharose 2B (product of Pharmacia Fine Chemicals) or the like.

The mRNA corresponding to the desired M-CSF can be isolated from the resulting mRNAs, concentrated and identified, for example, by fractionating the mRNAs by sucrose density gradient centrifugation, introducing the fractions into a protein translation system, such as Xenopus laevis oocytes, or rabbit reticulocyte lysates, wheat germ extracts or like cell-free system to cause the system to translate the fraction into a protein, and examining the protein for M-CSF activity, whereby the presence of the desired mRNA can be recognized. Instead of the determination of the M-CSF activity, an immunoassay using an antibody against M-CSF is also available in identifying the desired mRNA.

The purified mRNA thus obtained, which is usually unstable, is converted to a stable cDNA, which is then coupled to a replicon derived from a microorganism for the amplification of the desired gene. The conversion of the mRNA to the cDNA, i.e. the synthesis of the desired gene of the invention can be done generally in the following manner.

Using oligo dT as a primer (which may be free oligo dT or oligo dT as attached to a vector primer) and the mRNA as a template, a single-stranded cDNA complementary to the mRNA is synthesized therefrom in the presence of dNTP (dATP, dGTP, dCTP or dTTP) with use of a reverse transcriptase. The next step differs as follows depending on whether free oligo dT or oligo dT as attached to a vector primer is used.

In the former case, the mRNA used as a template is removed by alkaline hydrolysis, and a double-stranded DNA is synthesized from the single-stranded DNA serving as a template, using a reverse transcriptase or DNA polymerase. Subsequently, both ends of the double-stranded DNA are treated with exonuclease, a suitable linker DNA or a combination of bases amenable to annealing is attached to each end, and the resulting DNA is inserted into a suitable vector, such as EK-type plasmid vector or λgt phage vector.

In the latter case, a plasmid in the form of an open circle and having the same linker as above attached thereto, and a linker DNA (frequently used as such is a DNA fragment having a region which can be autonomously replicated in an animal cell and a mRNA transcription promotor) are annealed into a closed circle in the presence of the template mRNA. The mRNA is thereafter replaced by the DNA chain in the presence of dTNP and also in the presence of both RNase and DNA polymerase to obtain a complete plasmid DNA.

The DNA thus obtained is then introduced into a suitable host, such as E. coli, Bacillus subtilis or Saccharomyces cerevisiae, for transformation. The DNA can be introduced into the host by transformation, using a usual method, for example, by collecting cells primarily in the logarithmic growth phase, treating the cells with $CaCl_2$ to make them ready to accept the DNA and causing the cells to uptake in the plasmid. This method can be practiced in the presence of $MgCl_2$ or RbCl as is generally known to achieve an improved transformation efficiency. The host cells can be converted to spheroplasts or protoplasts before transformation.

From among the transformants thus prepared, the one harboring the cDNA with the desired M-CSF can be selected, for example, by one of the following methods.

(1) Screening with use of synthetic oligonucleotide probe When the amino acid sequence of the desired protein is wholly or partially has been elucidated (i.e. when a specific sequence of several amino acids in any region of the protein is known), oligonucleotides corresponding to the amino acids are synthesized in view of codon usage of the host cells. Thus synthesized oligonucleotides can be used either as a single or mixed probe. In the latter case, the number of combinations can be reduced by incorporating inosine. The oligonucleotide is labeled with $^{32}P$ or $^{35}S$, and hybridized with nitrocellulose filters having DNAs of the transformants fixed thereto. The desired transformant is selected from the positive hybrids obtained.

(2) Screening of animal cells producing M-CSF The transformants are incubated to amplify the desired genes, and animal cells are transfected with the amplified genes. (The plasmid to be used in this case is one autonomously replicatable and having a mRNA transcription promotor, or one that will integrate with an animal cell chromosome.) Thus, the cells are caused to produce proteins coded by the respective genes. The supernatant of each culture of such cells, or the cell extract is assayed for M-CSF activity, or M-CSF is detected using an antibody against M-CSF, whereby the desired transformant having the cDNA specifying M-CSF is selected.

(3) Selection using antibody against M-CSF cDNA is introduced into vectors which permit the transformant to express a protein. The desired transformant producing M-CSF is detected using an antibody against M-CSF and a second antibody against the antibody.

(4) Use of selective hybridization-translation system mRNAs from M-CSF producing cells are hybridized with the transformant-derived cDNA preblotted on the nitrocellulose filters, and the desired mRNA corresponding to the cDNA is recovered. The recovered mRNA is subjected to a protein translation system, such as Xenopus laevis oocytes, or rabbit reticulocyte lysates, wheat germ extracts or like cell-free system, and is thereby translated into protein. The protein is assayed for M-CSF activity, or is examined for the presence of M-CSF using an antibody against M-CSF to identify the desired transformant.

The DNA coding for M-CSF is obtained from the desired transformant by a known method, for example, by separating a fraction corresponding to the plasmid DNA from the cell, and isolating the cDNA region from the plasmid DNA.

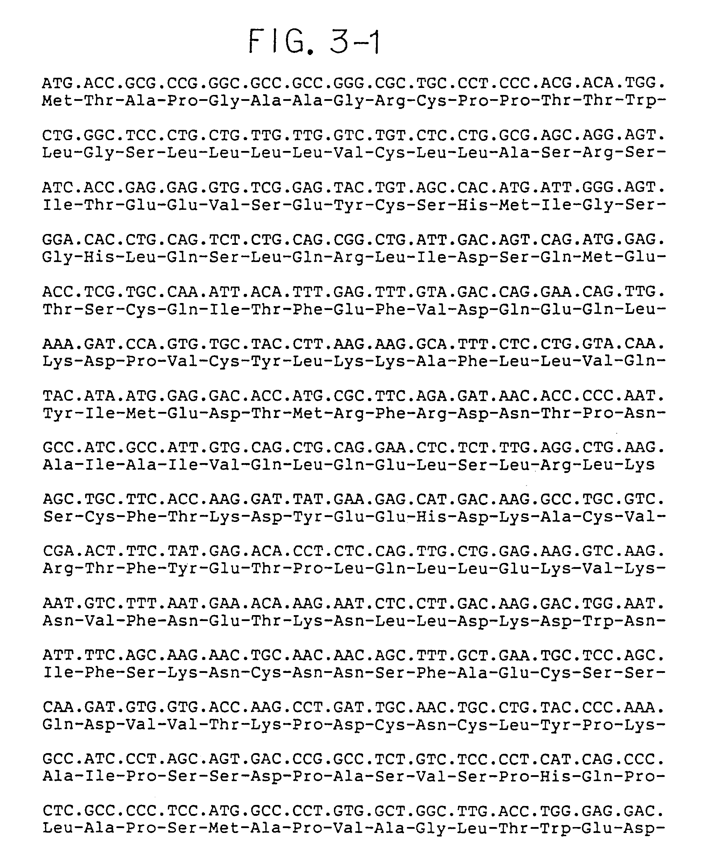

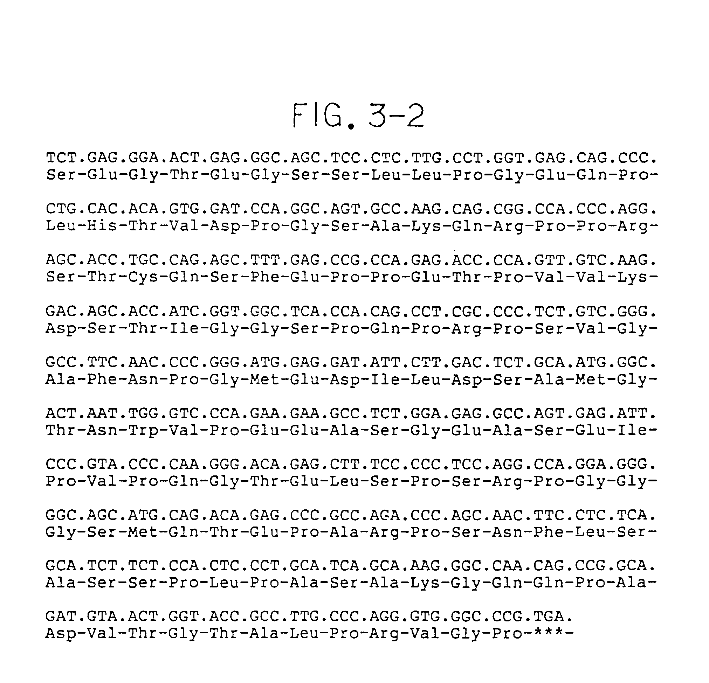

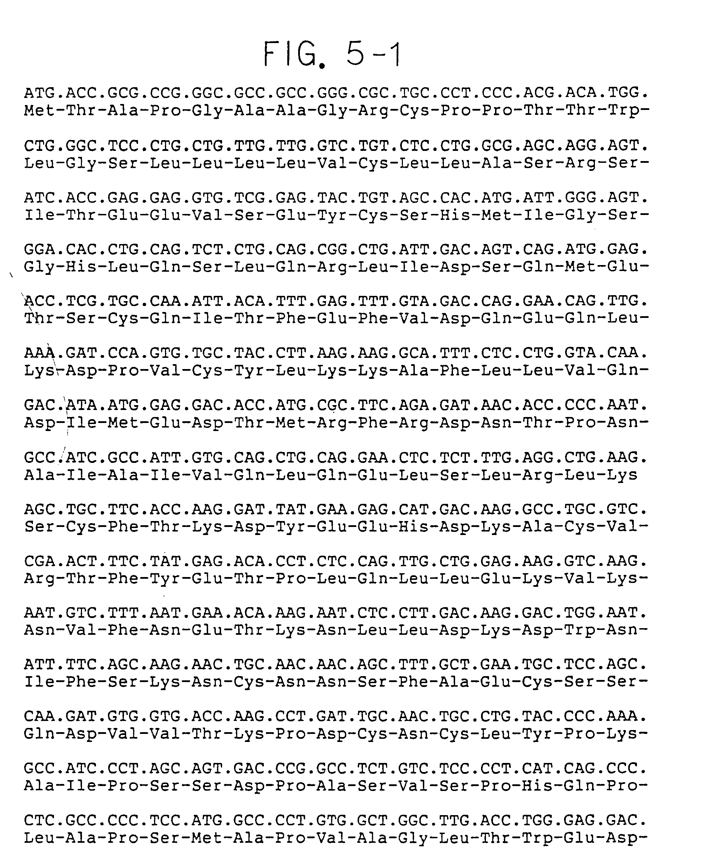

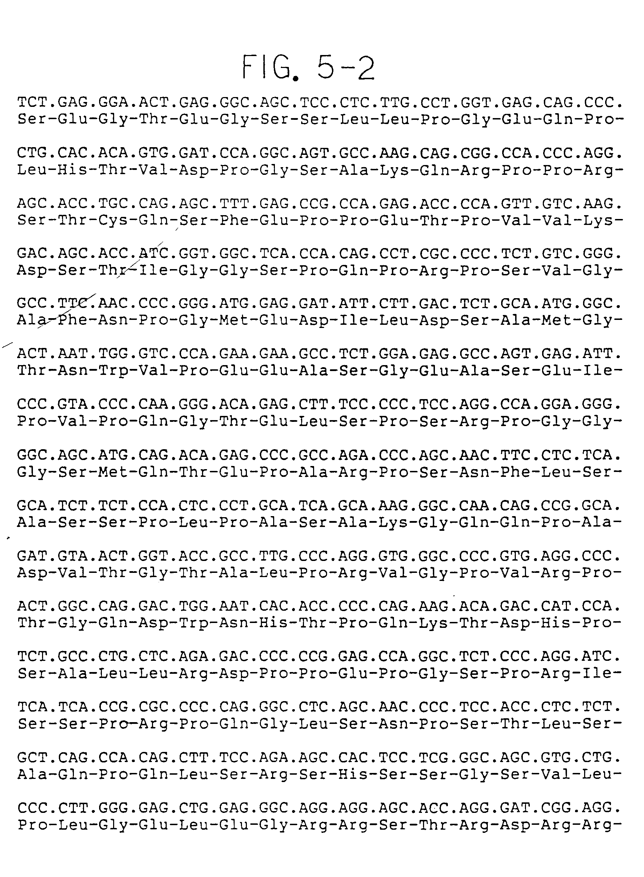

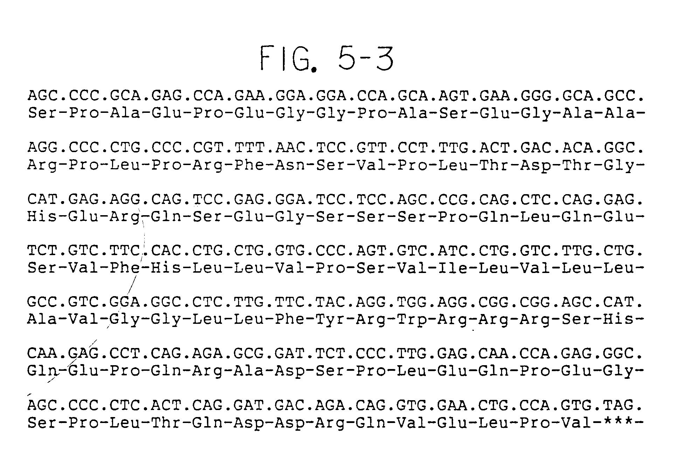

The DNA thus obtained is an example of the gene of the invention coding for a human M-CSF precursor which is defined by the sequence of 372 amino acids shown in Fig. 3 or the sequence of 554 amino acids shown in Fig. 5.

For the substance, obtained by genetic engineering techniques using the gene of the invention, to exhibit the biological activity of human M-CSF, the gene need not always have the above DNA, i.e. the DNA sequence coding for the entire amino acid sequence of the human M-CSF precursor. For example, DNAs coding for portions of the amino acid sequence are also included in the gene of the invention insofar as they permit exhibition of the biological activity of human M-CSF.

The fact that the entire amino acid sequence represented by the formula (1) is not always required for the exhibition of human M-CSF biological activity is apparent also from the fact that the precursor with the sequence of Fig. 3 has the 372nd amino acid (Pro) in the formula (1) at its C terminus, the fact that AGR-ON•CSF has the 35th amino acid (Val) at its N terminus, and the fact that the preparation of AGR-ON•CSF affords a by-product (minor component) which is a biological active M-CSF having the 33rd amino acid (Glu) or the 37th amino acid (Glu) at its N terminus. Further findings as to the similarity in the primary structure between hemopoietins appear to indicate that a naturally occurring M-CSF is present which has the 27th amino acid (Ala) at its N terminus (J.W. Schrader et al., Proc. Natl. Acad. Sci. U.S.A., Vol. 83, pp. 2458-2462 (1986)).

Further as shown in the example concerned and given later, it has been confirmed that the portion of the amino acid sequence of the formula (1) from the 178th amino acid (Cys) to the amino acid at the C terminus is not always necessary for the exhibition of the biological activity of human M-CSF.

Consequently, the gene of the invention is characterized in that the gene specifies a biologically active human M-CSF molecule and has a novel DNA sequence based on the information of the amino acid sequence shown in the formula (1). Stated more specifically, the gene of the present invention includes the DNA coding for the entire amino acid sequence represented by the formula (1), a DNA coding for the amino acid sequence of the formula (1) which is deficient in a portion thereof closer to the N terminus, e.g. in the sequence of the 1st to the 26th, the 1st to the 32nd, the 1st to the 34th or the 1st to the 36th amino acids, and/or in a portion thereof closer to the C terminus, e.g. in the whole or part of the sequence from the 178th amino acid to the C-terminus, and DNAs substantially equivalent to these DNAs.

The gene of the invention can also be prepared by a usual process for the chemical synthesis of nucleic acid, e.g. the phosphite triester process (Nature, 310, 105 (1984)), based on the above information. The present gene can further be prepared from the DNA coding for the polypeptide comprising the sequence of 372 amino acids shown in Fig. 3 or of 554 shown in Fig. 5 by a usual process. The latter method is especially convenient and suitable to practice.

When the gene is to be prepared from the DNA coding for the polypeptide comprising the sequence of 372 or 554 amino acids, usual procedures or means can be employed for the chemical synthesis of portions of the DNA, for the enzymatic treatments for cleaving, removing, adding or ligating DNA chains, and for the isolation and purification, or replication and selection, of the desired DNA. Thus, various known methods are available which are generally employed in the art for genes or DNA chains other than those of the invention. For example, the desired DNA can be isolated and purified by agarose gel electrophoresis, while codons in the nucleic acid sequence can be modified, for example, by site-specific mutagenesis (Proc. Natl. Acad. Sci., 81, 5662-5666 (1984)). The codon to be selected for the desired amino acid is not limited specifically but can be determined in a usual manner in view of the codon usage for the host cell to be utilized, etc.

The DNA sequence of the gene of the invention obtained by the above process can be determined and confirmed, for example, by the Maxam-Gilbert chemical modification method (Meth. Enzym., 65, 499-560 (1980)) or by the dideoxynucleotide chain termination method using M13 phage (Messing, J. and Vieira, J., Gene, 19, 269-276 (1982)).

Thus, human M-CSF can be prepared easily in a large quantity by recombinant DNA techniques using the gene of the invention.

With the exception of utilizing the specified gene of the invention, the human M-CSF can be prepared by common gene recombination techniques which are already known (Molecular Cloning, by T. Maniatis et al., Cold Spring Harbor Laboratory (1982); Science, 224, 1431 (1984); Biochem. Biophys. Res. Comm., 130, 692 (1985); Proc. Natl. Acad. Sci., U.S.A., 80, 5990 (1983); EP Laid-Open Pat. Appln. No.187991).

More specifically, the human M-CSF can be produced by preparing a recombinant DNA which can express the gene of the invention in host cells, transforming the DNA into the host cell and incubating the transformant.

Useful host cells can be either eucaryotic or procaryotic cells. The eucaryotic cells include cells of vertebrate animals, yeasts, etc. Generally used as cells of vertebrate animals are, for example, COS cells which are cells of monkey (Y. Gluzman, Cell, 23, 175-182 (1981)), dihydrofolate reductase defective strain of Chinese hamster ovary cell (G. Urlaub and L.A., Chasin, Proc. Natl. Acad. Sci., U.S.A., 77, 4216-4220 (1980)), etc., while useful cells are not limited to these cells. Useful expression vectors of vertebrate cells are those having a promotor positioned upstream of the gene to be expressed, RNA splicing sites, polyadenylation site, transcription termination sequence, etc. These vectors may further have a replication origin when required. Examples of useful expression vectors include pSV2dhfr having an initial promotor of SV40 (S. Sabramani, R. Mulligan and P. Berg. Mol. Cell. Biol., 1, 854-764), which is not limitative.

Yeasts are widely used as eucaryotic microorganisms, among which those of the genus Saccharomyces are generally usable. Examples of popular expression vectors of yeasts and like eucaryotic microorganisms include pAM82 having a promotor for acid phosphatase gene (A. Miyanohara et al., Proc. Natl. Acad. Sci., U.S.A., 80, 1-5 (1983), etc.

E. coli and Bacillus subtilis are generally used as procaryotic hosts. The present invention employs, for example plasmid vectors capable of replication in the host. To express the gene of the invention in the vector, expression plasmids can be used which have a promotor and SD (Shine-Dalgarno) sequence upstream to the present gene and ATG required for initiating protein synthesis. Widely used as host E. coli is E. coli K12 strain. pBR322 is a vector which is generally used. However, these are not limitative, and various known strains and vectors are usable. Examples of promotors usable are tryptophan promotor, $P_L$ promotor, lac promotor, lpp promotor, etc. The gene can be expressed with use of any of these promotors.

With reference to the case wherein COS cells are used as the host, the expression vector that can be used is one having SV40 replication origin, capable of autonomously replicating in the COS cell and having a transcription promotor, transcription termination signal, RNA splicing site, etc. For example, when plasmid pcDE is used as shown in the example to follow, the gene of the invention is attached to the plasmid at the site of restriction enzyme EcoRI positioned downstream from SV40 early promotor, whereby the desired expression plasmid can be obtained.

The desired recombinant DNA thus obtained can be introduced into the host cell by a common method. For example, the expression plasmid prepared by inserting the desired gene into the plasmid pcDE can be introduced into COS cells by the DEAE-dextran method or calcium phosphate-DNA coprecipitation method, consequently readily giving the desired transformant.

The desired transformant can be incubated by a usual method, whereby biologically active human M-CSF is produced and accumulated. The medium to be used for the incubation is one suitably selected from among those usually used for the host cell employed. Examples of media usable for COS cells are RPMI-1640 medium, Dulbecco's modified Eagle's MEM, etc. which may be supplemented with fetal calf serum (FCS) or like serum component when so required.

The M-CSF produced by the transformant intra- or extra-cellularly can be separated off and purified by various separation procedures utilizing the physical or chemical properties of the product. (See for example, "Biological Data Book II," pp. 1175-1259, 1st edition, 1st print, June 23, 1980, published by Kabushiki Kaisha Tokyo Kagakudojin.) Examples of useful procedures are treatment with use of a usual protein precipitating agent, ultrafiltration, molecular sieve chromatography (gel filtration), adsorption chromatography, ion-exchange chromatography, affinity chromatography, high-performance liquid chromatography (HPLC) and like liquid chromatography, dialysis, and combinations of such procedures.

According to a preferred method of separation, the desired substance is partially purified, from the culture supernatant. This partial purification is conducted, for example, by a treatment using a salting-out agent such as ammonium sulfate, sodium sulfate or sodium phosphate and/or ultrafiltration using a dialysis membrane, flat membrane, hollow fiber membrane or the like. These treatments are conducted in the same manner as usually done under usual conditions.

The roughly purified product thus obtained is then subjected to adsorption chromatography, affinity chromatography, gel filtration, ion-exchange chromatography, reverse-phase chromatography or the like, or a combination of such procedures to obtain the desired homogeneous substance.

Adsorption chromatography can be conducted using, for example, phenyl-Sepharose, octyl-Sepharose or like carrier.

Affinity chromatography can be practiced by chromatography utilizing, for example, ConA-Sepharose, Lentil lectin-Sepharose (product of Pharmacia) or like resin, when so desired.

Gel filtration can be practiced using an agent which is made of dextran gel, polyacrylamide gel, agarose gel, polyacrylamide-agarose gel, cellulose or the like. Examples of useful agents commercially available are Sephadex G type, Sepharose type, Sephacryl type (all products of Pharmacia), Cellulofine (product of Chisso Corporation), Biogel P type, Biogel A type (both products of Bio Rad Lab.), Ultrogel AcA (product of LKB), TSK-G type (product of Toyo Soda Mfg. Co., Ltd.), etc.

Ion exchange chromatography can be conducted by chromatography using an anionic ion exchange material, for example with diethylaminoethyl (DEAE) or the like serving as the exchange group.

Reverse-phase chromatography can be conducted using a carrier which comprises a substrate of silica gel or the like having coupled thereto a functional group such as $C_1$, $C_3$ or $C_4$ alkyl, cyanopropyl or phenyl. More specifically, the chromatography can be conducted with $C_4$ Hi-Pore Reverse-Phase HPLC Column (RP-304, Bio-Rad Laboratories) using acetonitrile, trifluoroacetic acid (TFA), water or the like, or a mixture of such solvents as the eluent.

By the processes described above, the desired M-CSF can be commercially produced with high purity in high yields.

For use as a drug, the M-CSF thus prepared is formulated into pharmacological compositions containing an effective amount of M-CSF and a usual pharmacologically acceptable nontoxic carrier. The composition is given via a route of administration suited to the form of the composition. Such compositions are, for example, in the form of liquid preparations including solution, suspension, emulsion and the like, which are given usually orally, intravenously, subcutaneously, intracutaneously or intramuscularly. Such forms or methods of administration are not limited specifically; the M-CSF composition can be prepared in various forms usually used and suited, for example, to oral or nonoral administration. The composition can be provided also as a dry preparation which can be reconstituted to a liquid for use by addition of a suitable carrier. While the amount of the composition to be given in any form is not limited specifically but can be determined suitably according to the desired pharmacological effect, the kind of disease, the age and sex of the patient, the degree of disease, etc., the composition is administered usually at a dose of about 0.001 to about 1 mg/kg/day calculated as the amount of the active component, i.e. M-CSF, in terms of protein amount. The daily dose is given singly or dividedly.

The process for preparing the gene of the invention, the process for preparing M-CSF with use of the gene, the features of the invention, etc. will be described in greater detail with reference to the following examples.

The specimens prepared in these examples were assayed for CSF activity by the following method.

Method of determining CSF activity

Fetal calf serum (FCS, 20 ml), 30 ml of $\alpha$-medium and 20 ml of $\alpha$-medium of 2-fold concentration are mixed together, and the solution is then maintained at 37°C. A 23.3-ml portion of the solution is admixed with 10 ml of 1% solution of agar (product of Difco Laboratories) already maintained at 50°C to obtain an agar medium, which is then maintained at 37°C.

Bone marrow cells (BMC) collected from the femur of a mouse of BALB/C strain are washed with Hanks solution twice and thereafter suspended in $\alpha$-medium to a concentration of $10^7$ cells/ml, and 1 ml of the suspension added to the agar medium maintained at 37°C. The mixture is thoroughly stirred and then maintained at 37°C. A 0.5-ml portion of the mixture is placed into wells (Tissue Culture Cluster 12, product of Costar Corporation) already containing 50 $\mu$l of a test sample, and the resulting mixture is quickly stirred and then allowed to stand at room temperature. On solidification of the mixture in each well, the wells are placed into a $CO_2$ incubator and incubated at 37°C for 7 days.

The number of colonies thus produced is counted under an optical microscope to provide an index for the CSF activity. When observed morphologically, the colonies formed were all macrophage colonies.

The accompanying drawings are as follows.

Fig. 1 is a restriction enzyme map of cDNA of λcM5;

Fig. 2 (Figs. 2-1 to 2-4) shows the nucleotide sequence of the cDNA as determined by the Maxam-Gilbert chemical modification method and the dideoxynucleotide chain termination method using M13 phage;

Fig. 3 (Figs. 3-1 and 3-2) shows the primary structure of M-CSF precursor protein coded for by the cDNA;

Fig. 4 (Figs. 4-1 to 4-4) shows the nucleotide sequence of the cDNA of λcM11;

Fig. 5 (Figs. 5-1 to 5-3) shows the primary structure of M-CSF precursor protein coded for by the λcM11 cDNA;

Fig. 6 is a diagram showing a procedure for preparing COS cell expression vector pcDE;

Fig. 7 is a diagram showing a procedure for preparing M-CSF expression plasmid pcDM•CSF;

Fig. 8 is a diagram showing a procedure for preparing M-CSF expression plasmid pcDM•CSF11-185;

Fig. 9 (Figs. 9-1 to 9-3) is a diagram showing a procedure for preparing M-CSF expression plasmid pIN-III(lpp$^p$-5)-OmpA-MCSF11-NV151;

Fig. 10 shows the amino acid sequence coded for by the M-CSF expression plasmid pIN-III(lpp$^P$-5)-OmpA-MCSF11-NV151;

Fig. 11 shows the amino acid sequence coded for by M-CSF expression plasmid pIN-III(lpp$^P$-5)-OmpA-MCSF11-NV143; and

Fig. 12 (Figs. 12-1 to 12-2) is a diagram showing a procedure for preparing M-CSF expression plasmid pcDM•CSF11-dhfr.

Example 1

Preparation of gene of the invention

(1) Incubation of AGR-ON cells

Cultured human T cell line AGR-ON (ATCC deposition No.CRL-8199) was suspended to a concentration of about $10^5$ cells/ml in RPMI-1640 medium (product of Flow-Lab. Inc.) containing 10% newborn calf serum (NCS), 20 mM N-2-hydroxyethylpiperazine-N'-2-ethanesulfonic acid (HEPES), 100 $\mu$g/ml streptomycin, 100 units/ml of penicillin G, 50 $\mu$g/ml of gentamycin, 5 x $10^{-5}$ M 2-mercaptoethanol and 1 mM glutamine. A one-liter portion of the suspension was incubated in five 200-ml tissue culture flasks (product of Corning) at 37°C for 72 hours.

(2) Extraction of mRNA

About 5 x $10^8$ AGR-ON cells obtained by the procedure (1) were dissolved in 50 ml of 4 M guanidine thioisocyanate solution (4 M guanidine isothiocyanate, 50 mM Tris-HCl (pH 7.6), 10 mM EDTA, 2% Sarkosyl and 140 mM 2-mercaptoethanol), and while heating the solution at 60°C, the DNA was sheared using 50-ml syringe having G18G injection needle.

To one part by volume of the solution were added 1 part by volume of phenol, 0.5 part by volume of 100 mM sodium acetate (pH 5.2)-10 mM Tris-HCl (pH 7.4)-1 mM EDTA solution, and 1 part by volume of chloroform-isoamyl alcohol (24:1) mixture, as heated to 60°C. The mixture was shaken in a water bath at 60°C for 10 minutes and then centrifuged at 4°C at 3000 r.p.m. for 15 minutes. The aqueous layer was separated off and extracted with phenol-chloroform twice and further with chloroform twice. To the combined extract was added cold ethanol in twice the volume of the extract. The mixture was maintained at -20°C for 60 minutes and centrifuged at 4°C at 3000 r.p.m. for 20 minutes. The RNA precipitate thus obtained was dissolved in 50 ml of 100 mM Tris-HCl (pH 7.4)-50 mM NaCl-10 mM EDTA-0.2% SDS solution, and proteinase K (product of Merk) was added to the solution to a concentration of 200 $\mu$g/ml, followed by reaction at 37°C for 60 minutes. At 60°C, the reaction mixture was extracted with phenol-chloroform twice and further with chloroform twice. To the combined extract were added 3 M sodium acetate (pH 5.2) in 1/10 the volume of the extract and cold ethanol in twice the volume thereof. The mixture was allowed to stand at -70°C for 60 minutes and then centrifuged at 4°C at 3000 r.p.m. for 20 minutes. The RNA precipitate was washed with cold 70% ethanol and thereafter dissolved in TE solution (10 mM Tris-HCl (pH 7.5) and 1 mM EDTA).

In this way, about 5 mg of total RNA was obtained from the 5 x $10^8$ AGR-ON cells.

To obtain mRNA from the total RNA, the RNA was subjected to column chromatography using oligo-(dT)cellulose (Collaborative Research Inc.). For adsorption, 10 mM Tris-HCl (pH 7.5)-1 mM EDTA-0.5 M NaCl solution was used. The column was washed with the same solution and with 10 mM Tris-HCl (pH 7.5)-1 mM EDTA-0.1 M NaCl solution, and the RNA was then eluted with 10 mM Tris-HCl (pH 7.5)-1 mM EDTA.

The above procedure gave about 110 $\mu$g of mRNA.

(3) Preparation of cDNA library

cDNA was prepared from a 5-$\mu$g portion of the mRNA obtained by the procedure (2), using cDNA Synthesis System (product of Amersham).

The cDNA (about 0.6 $\mu$g) obtained was dried in a vacuum and then dissolved in 20 $\mu$l of 50 mM Tris-HCl (pH 7.5)-10 mM EDTA-50 mM DTT-40 $\mu$M S-adenosyl-L-methionine solution. After addition of 16 units of EcoRI methylase (product of New England Biolab.), the solution was reacted at 37°C for 15 minutes.

The reaction mixture was heated at 70°C for 10 minutes to terminate the reaction and then extracted with phenol-chloroform. To the extract were added 3 M sodium acetate (pH 5.2) in 1/10 the volume of the extract and ethanol in 2.5 times the volume thereof, and the mixture allowed to stand at -70°C for 15 minutes. The mixture was centrifuged at 4°C at 15000 r.p.m. for 15 minutes, the DNA precipitate dissolved in 10 $\mu$l of 50 mM Tris-HCl (pH 7.5)-10 mM MgCl$_2$-10 mM DTT-1 mM ATP-100 $\mu$g/ml of EcoRI linker (5'-GGAATTCC-3', product of Takara Shuzo Co., Ltd., Japan), 350 units of T4DNA ligase

10

(product of Takara Shuzo Co., Ltd.) added to the solution, and the mixture reacted at 14°C for 16 hours.

The reaction mixture obtained was heated at 70°C for 10 minutes to terminate the reaction. To the mixture were added 16 $\mu$l of 100 mM NaCl-50 mM Tris-HCl (pH 7.5)-7 mM MgCl$_2$-10 mM DTT solution and 40 units of the restriction enzyme EcoRI (Takara Shuzo Co., Ltd.), and the mixture was maintained at 37°C for 3 hours for digestion.

To the reaction mixture was added 0.8 $\mu$l of 0.5 M EDTA (pH 8.0) to terminate the reaction, and an excess of EcoRI linker was removed by Biogel A50m (product of Bio-Rad Laboratories) column chromatography. To the cDNA fraction was added 1 $\mu$g of $\lambda$gt11DNA (Protoclone GT, Promega Biotec.) digested with the restriction enzyme EcoRI and treated with alkaline phosphatase to remove the 5'-phosphate group. Also added to the fraction were 3 M sodium acetate (pH 5.2) in 1/10 the volume of the fraction and ethanol in 2.5 times the volume thereof. The mixture was allowed to stand at -70°C for 30 minutes and centrifuged at 4°C at 15000 r.p.m. for 15 minutes. The DNA precipitate was washed with 70% ethanol, then dried in a vacuum and dissolved in 7 $\mu$l of water.

After addition of 2 $\mu$l of 500 mM Tris-HCl (pH 7.5)-100 mM MgCl$_2$, the solution was subsequently maintained at 42°C for 15 minutes and thereafter cooled to room temperature. To the solution were then added 1 $\mu$l of 100 mM DTT, 1 $\mu$l of 10 mM ATP and 175 units of T4DNA ligase. The mixture was incubated at 14°C for 16 hours.

To 10 $\mu$l of the reaction mixture was added a $\lambda$ phage packaging extract (Packagene System, Promega Biotec.), and the mixture was maintained at 22°C for 2 hours, whereby recombinant phage DNA was packaged in vitro.

After addition of 0.5 ml of phage dilution buffer (100 mM NaCl-10 mM Tris-HCl (pH 7.9)-10 mM MgSO$_4$•7H$_2$O) and 25 $\mu$l of chloroform, the resulting solution was stored at 4°C.

(4) Screening of cDNA library

(4)-1. Preparation of synthetic probe

The following sequence was used for a synthetic probe based on the information of the amino terminal sequence (27 residues) of AGR-ON•CSF purified from an AGR-ON culture supernatant.

```
      Val – Ser – Glu – Tyr – Cys – Ser – His

    5' GTG   TCG   GAG   TAC   TGT   AGC   CAC 3'

    3' CAC   AGC   CTC   ATG   ACA   TCG   GTG 5'
```

A nucleotide sequence (shown immediate above) complementary to the sequence of the foregoing formula was synthesized by the following procedure in order to use as a probe for selecting a recombinant phage having cDNA coding for M-CSF.

The desired completely protected DNA was synthesized by the solid-phase phosphite triester process (Nature, 310, 105 (1984)) wherein an N,N-dialkylmethylphosphoroamidite derivative is used as the condensation unit, using an automatic synthesizer (380A DNA synthesizer, Applied Biosystems Inc.). The completely protected DNA was then treated with 28% of ammonia water at 55°C for 10 hours, whereby the protective groups (i.e. acyl groups on the amino groups of A, G and C) other than the DMTr (dimethoxytrityl) serving as a protective group and attached to the OH at the 5' terminus were removed to give partially protected DNA (to be referred to as "DMTr unit"). The DMTr unit was then purified by reverse-phase high-performance liquid chromatography (HPLC) using a ODS (product of Yamamura Kagaku Kenkyusho Co., Ltd., Japan) column and thereafter treated with 80% acetic acid at room temperature for 20 minutes to obtain a crude oligonucleotide. The product was further purified by reverse-phase HPLC with the ODS column to prepare the desired oligonucleotide.

The DNA (0.8 $\mu$g) obtained was reacted with 18 units of T4 polynucleotide kinase (Takara Shuzo Co., Ltd.) at 37°C for 1 hour in 100 $\mu$l of a reaction medium (50 mM Tris-HCl (pH 7.6), 10 mM MgCl$_2$, 10 mM 2-mercaptoethanol, 200 $\mu$Ci[$\gamma$-$^{32}$P]-ATP) to label the 5' end of the DNA with $^{32}$P. To separate the labeled DNA from the unreacted [$\gamma$-$^{32}$P]ATP, the reaction mixture was subjected to gel filtration using Biogel P-30 (Bio-Rad Laboratories). The labeled DNA fractions were pooled and preserved at -20°C. The probe obtained was at least 10$^8$ cpm/$\mu$g DNA in specific radioactivity.

(4)-2 Plaque hybridization

E. coli Y1090 strain was inoculated in 40 ml of LB medium (10 g of bacto-tripton, 5 g of bacto-yeast extract and 5 g/l of NaCl) containing 50 $\mu$g/ml of ampicillin and 0.2% of maltose and incubated in a 300-ml flask overnight at 37°C. The culture was centrifuged at 4°C at 3000 r.p.m. for 15 minutes to collect cells. The cell pellets were suspended in 20 ml of SM medium (100 mM NaCl-50 mM Tris-HCl (pH 7.5)-10mM MgSO$_4$•7H$_2$O-0.01%gelatin) and stored at 4°C.

To a 0.3 ml portion of the cell suspension was added 0.1 ml of the recombinant phage solution which was obtained by the procedure (3) and diluted to 3.5 x 10$^5$ pfu (plaque forming units)/ml with SM medium, and the mixture was then incubated at 37°C for 15 minutes. Subsequently, LB soft agar medium (10 g of bacto-tripton, 5 g of yeast extract, 5 g of NaCl and 7 g/liter of agarose) prewarmed to 47°C was admixed with the mixture, and the resulting mixture was overlayered onto LB agar plate (10 g of bactotripton, 5 g of bacto-yeast extract, 5 g of NaCl and 15 g/liter of bacto-agar), and incubated at 42°C overnight. Same procedure was repeated in each of 20 petri dishes.

A nylon filter (BNRG 132, Pall Ultrafine Filtration Corp.) having a diameter of 132 mm was placed over each agar plate having a plaque formed thereon to prepare a replica filter. The agar plate was preserved at 4°C as a master plate.

The filter was treated with 0.5 M NaOH-1.5 M NaCl, then with 0.5 M Tris-HCl (pH 7.5)-1.5 M NaCl and thereafter with 0.3 M NaCl-0.02 M NaH$_2$PO$_4$ (pH 7.4)-0.002 M EDTA (pH 7.4), dried in air and baked under vacuum at 80°C for 1 hour.

The baked filter was maintained with gentle shaking at 42°C for 6 hours in 50 ml of 0.75 M NaCl-0.075 M sodium citrate-10 mg/ml of Ficoll-10 mg/ml of polyvinylpyrrolidone-10 mg/ml BSA-10 mM sodium phosphate (pH 6.5)-0.2% SDS-0.1 mg/ml salmon sperm DNA. Subsequently, the filter was placed into the same solution as above having the probe added thereto at a concentration of 10$^6$ cpm/ml and hybridized at 42°C for 20 hours with gentle shaking.

After the hybridization, the filter was withdrawn from the solution, washed with 0.9 M NaCl-0.09 M sodium citrate at room temperature three times and then with the same solution at 56°C for 5 minutes.

The filter was dried in air and thereafter autoradiographed at -70°C for 2 days on an X-ray film (XR5, Eastman Kodak Co.) using sensitized paper.

After developing the film, the plaques corresponding to the signal region were scraped off the master plate, and the above procedure is repeated to purify the plaques having a positive signal, whereby positive clone λcM5 and λcM11 were eventually isolated.

(5) Structural analysis of the cDNA clone

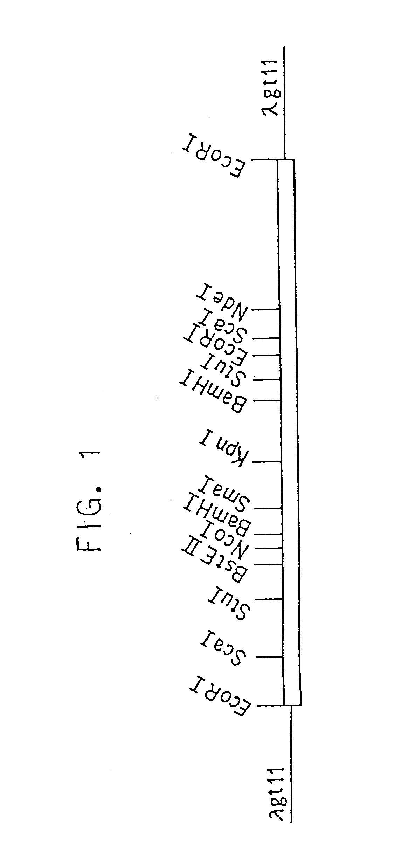

Restriction enzyme analysis of λcM5 was carried out, and the result is shown in Fig. 1.

As shown in Fig. 1, the cDNA is about 2.5 kb in entire length and has sites of cleavage by BstEII (product of New England Biolab.), NcoI (product of Takara Shuzo Co., ltd.), SmaI (Takara), KpnI (Takara), EcoRI (Takara) and NdeI (product of New England Biolab.), one cleavage site for each, and two sites of cleavage by each of ScaI, StuI and BamHI (all products of Takara).

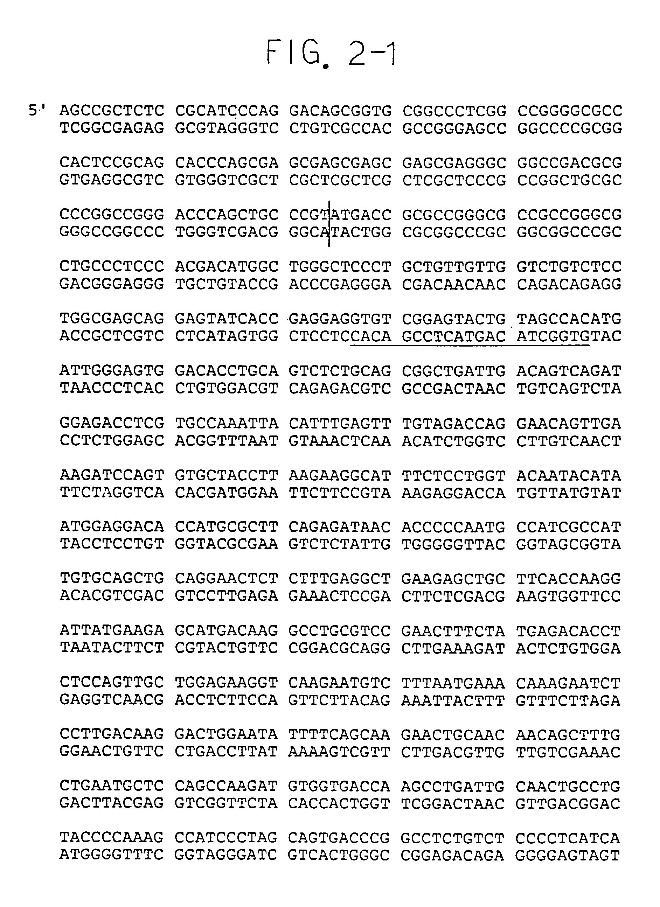

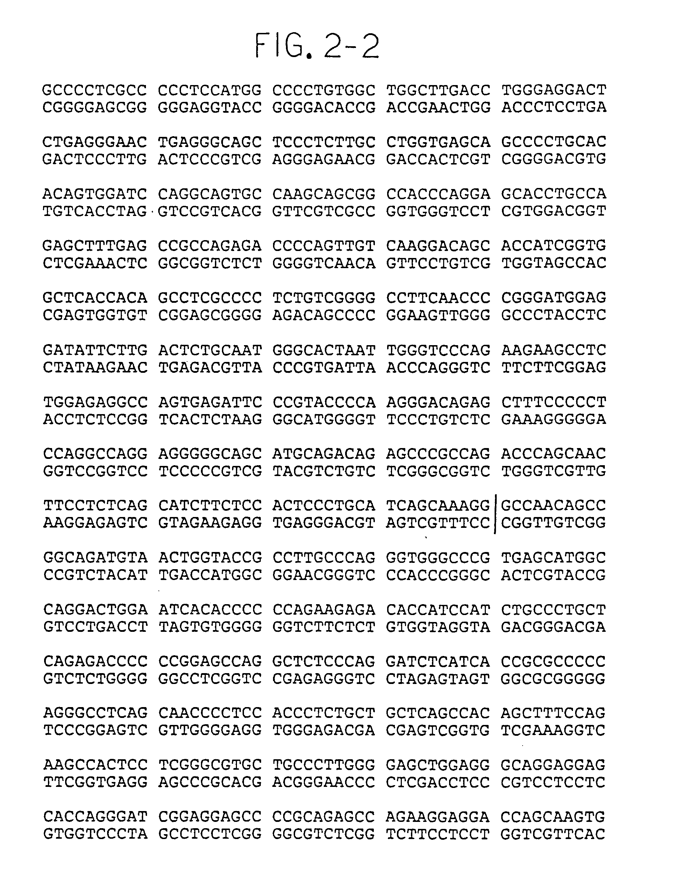

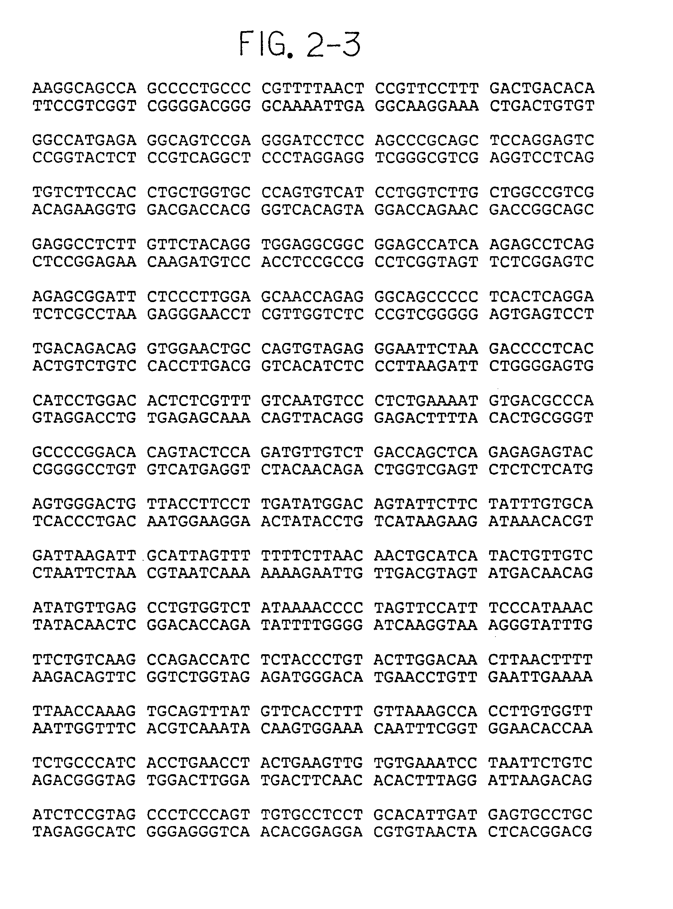

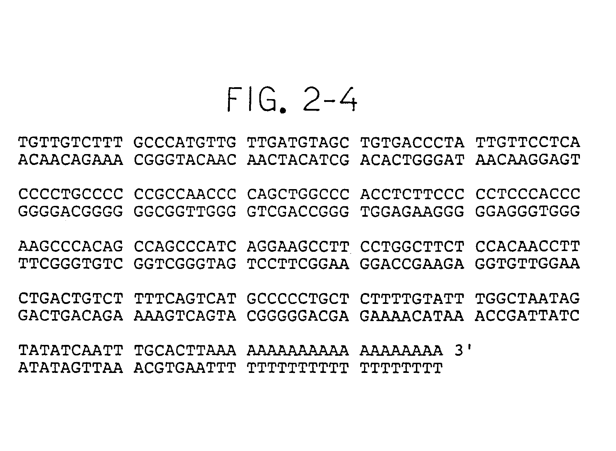

Next, the nucleotide sequence of the cDNA was determined by the Maxam-Gilbert chemical modification method and the dideoxynucleotide chain termination method using M13 phage. Fig. 2 (Figs. 2-1 to 2-4) shows the result.

Fig. 2 reveals that the region (shown as underlined) of the 227th to the 247th bases from the 5' terminus is complementary to the synthetic probe.

When the cDNA of λcM5 is searched for the longest reading frame, the region of the 125th to the 1240th bases from the 5' terminus was found to be this frame. The amino acid sequence corresponding to the sequence of the 227th to 307th bases and coded by the codons thereof was completely in match with the 27 amino acids at the N terminal region of AGR-ON•CSF. This indicates that the cDNA of λcM5 is the cDNA coding for an M-CSF precursor protein.

Fig. 3 (Figs. 3-1 and 3-2) shows the primary structure of the M-CSF precursor protein predicted from these results.

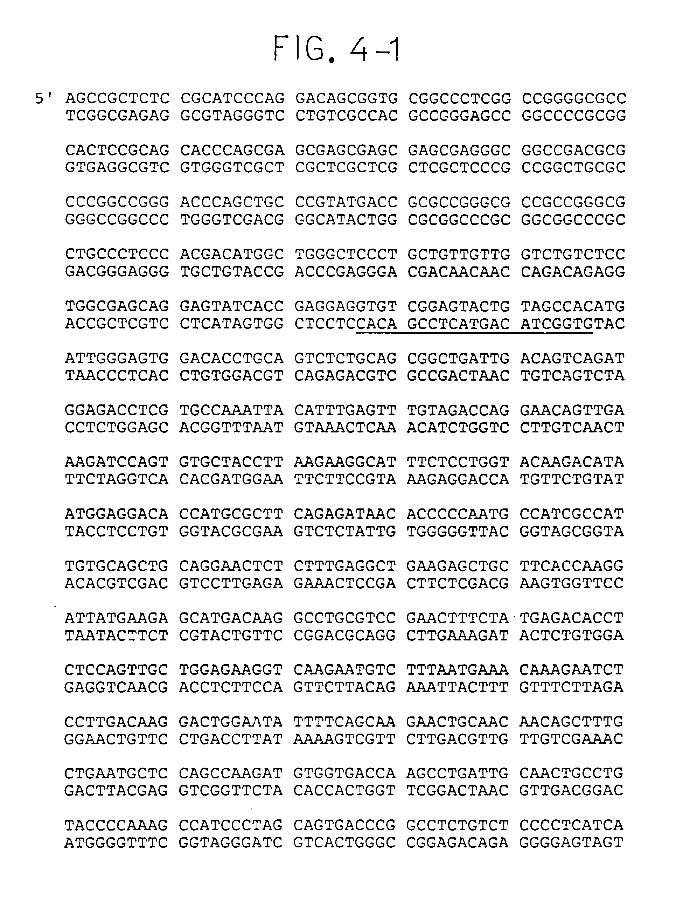

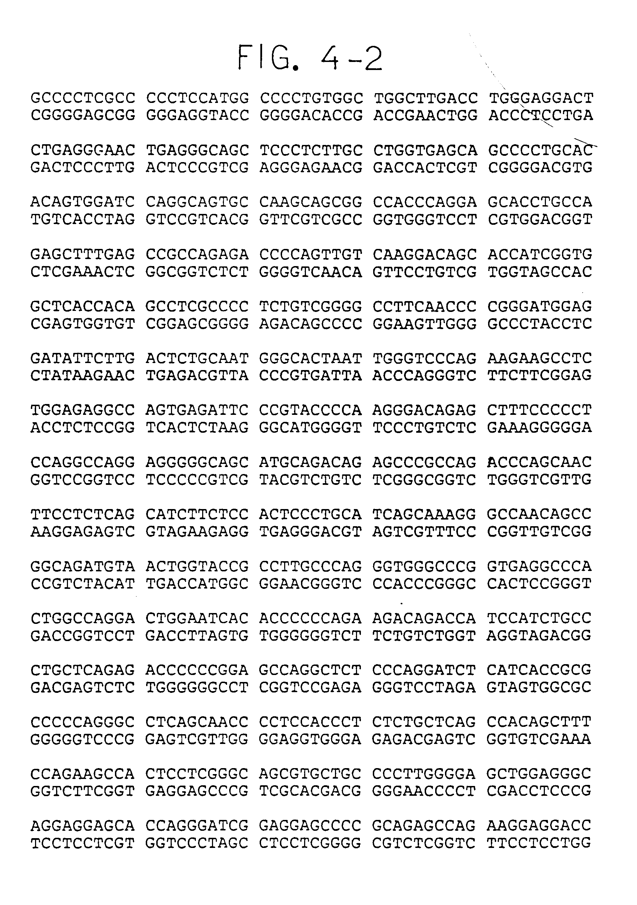

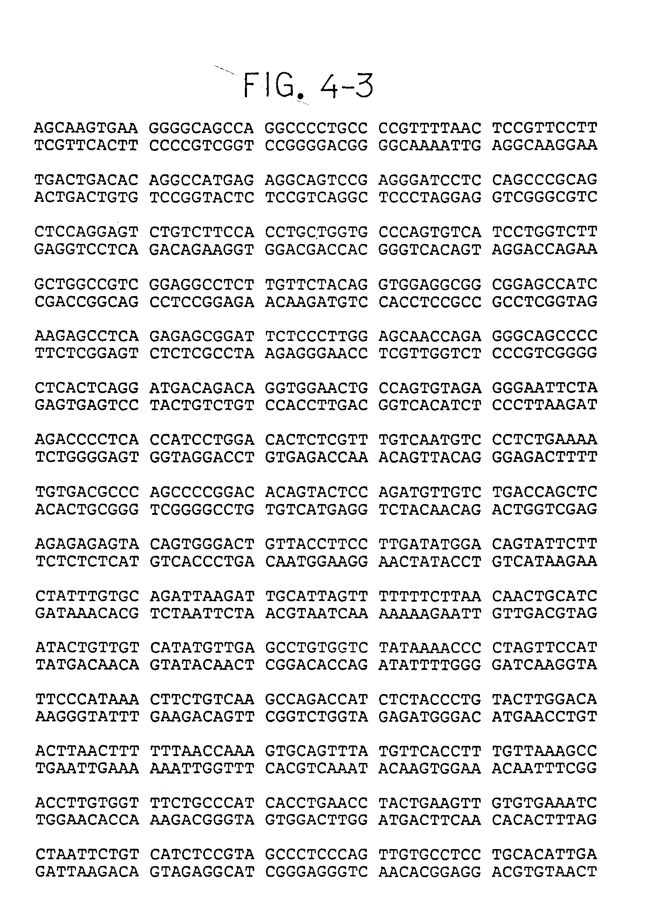

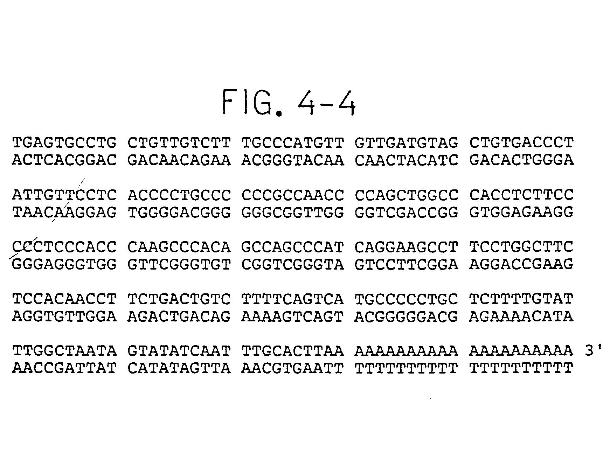

Fig. 4 (Figs. 4-1 to 4-4) shows the nucleotide sequence of the cDNA of λcM11 determined in the same manner as above and coding for an M-CSF precursor protein, the predicted primary structure of which is shown in Fig. 5 (Figs. 5-1 to 5-3).

Example 2

Preparation of recombinant M-CSF (r-MCSF) in COS cells

COS-1 cell used in this example is a cell line which is obtained by transforming monkey renal cell line, CVI, with replication origin (Ori)-deficient SV40, and thereby made to express SV40 early gene and rendered T angigen positive (Cell, 23, 175-182 (1981)).

(1) Preparation of COS cell expression vector pcDE

Plasmid pcDVI (H. Okayama and P. Berg, Mol. Cell. Biol., 3, 280-289 (1983)) was first cleaved with restriction enzyme KpnI, and projections at the 5' and 3' termini were removed by T4 DNA polymerase (product of BRL) to form blunt ends.

On the other hand, the 5' end of EcoRI linker (5'-GGAATTCC-3') (product of Takara Shuzo Co., Ltd.) was phosphorylated with T4 polynucleotide kinase and ligated to the blunt-ended DNA fragment using T4 DNA ligase. The resultant ligate is cleaved with restriction enzyme EcoRI and further with restriction enzyme HindIII. The reaction product obtained was subjected to agarose gel electrophoresis, whereby EcoRI-HindIII DNA fragment of 2590 bp was isolated and purified.

Plasmid pL1 (H. Okayama and P. Berg, Mol. Cell. Biol., 3, 280-289 (1983)) was cleaved with restriction enzyme PstI (product of Takara Shuzo Co., Ltd.) and the 5' and 3' ends were converted to blunt ends using T4 DNA polymerase. In the same manner as above, EcoRI linker was ligated to the DNA fragment, the resulting DNA fragment was cleaved with restriction enzyme, and the reaction product was subjected to agarose gel electrophoresis, whereby EcoRI-HindIII DNA fragment with 580 bp was isolated and purified.

The DNA fragment obtained was ligated to the EcoRI-HindIII DNA fragment previously prepared, using T4 DNA ligase, to afford the desired plasmid pcDE.

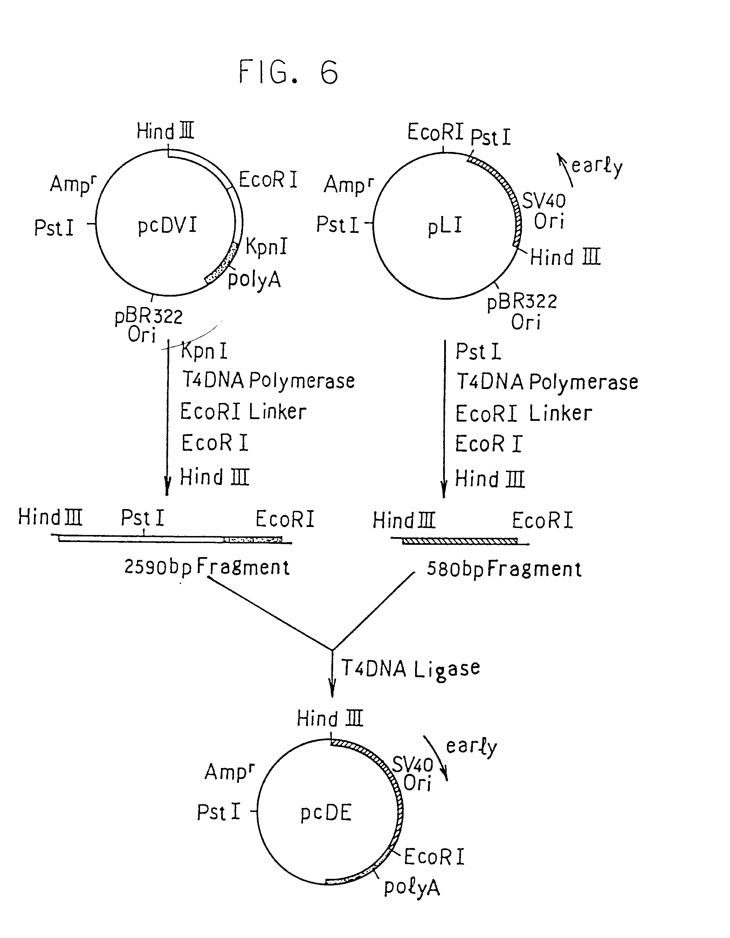

Fig. 6 schematically shows the above procedure.

(2) Preparation of M-CSF expression plasmid pcDM•CSF

λcM5 DNA was partially digested with restriction enzyme EcoRI, and size-fractionated on agarose gel to isolate and purify a cDNA fragment (about 2.5 kb) as a partially digested EcoRI fragment.

The COS cell expression vector pcDE obtained by the procedure (1) was cleaved with restriction enzyme EcoRI and ligated to the cDNA fragment prepared above, using T4 DNA ligase, whereby the desired plasmid pcDM•CSF was obtained.

The plasmid thus obtained was introduced into E. coli HB101 strain by transformation. The desired transformant was selected by the restriction enzyme analysis of the plasmid DNA obtained according to the alkaline lysis method (T. Maniatis et al., Molecular Cloning, pp. 90, Cold Spring Harvor Laboratory (1982)).

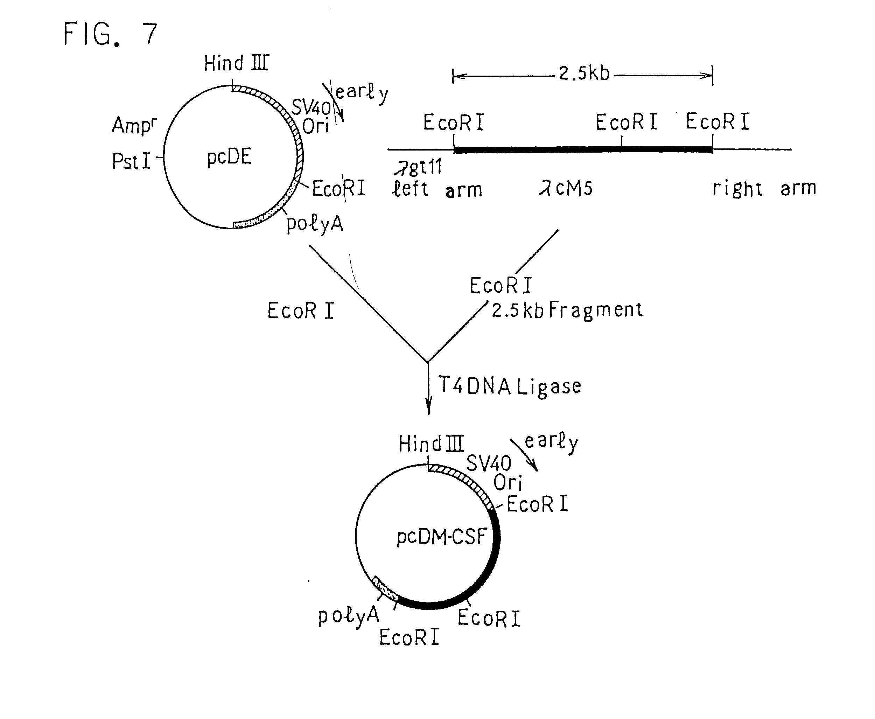

Fig. 7 schematically shows the above procedure.

(3) Preparation of r-MCSF

COS-1 cells were transfected with the pcDM•CSF prepared by the procedure (2), by the DEAE-dextran method (Proc. Natl. Acad. Sci. U.S.A., Vol. 81, p1070 (1984)) and tested for the production of r-MCSF by the following procedure.

COS-1 cells were first suspended to a concentration of about 2.5 x $10^5$ cells/ml in RPMI-1640 medium supplemented with 10% fetal calf serum (FCS). The suspension was placed into Tissue Culture Cluster-6 (product of Costar Corp.) in an amount of 2 ml in each well and incubated overnight at 37°C in a $CO_2$ incubator.

Subsequently, the medium was removed, the cells were washed with RPMI-1640 medium twice, 1 ml of RPMI-1640 (containing 50 mM Tris-HCl (pH 7.4) and 400 $\mu$g/ml DEAE-dextran (Pharmacia)) containing 10 $\mu$g of pcDM•CSF obtained by the procedure (2) was then added to the cells, and the mixture was allowed to stand in a $CO_2$ incubator for 4 hours. After removing the medium, the cells were washed twice with RPMI-1640 and then incubated for 3 hours with addition of 3 ml of RPMI-1640 medium containing 150 $\mu$M chloroquine. The medium was subsequently removed, and the cells were washed with RPMI-1640 and thereafter incubated in 10% FCS-containing RPMI-1640 medium at 37°C for 72 hours in a $CO_2$ incubator.

The culture supernatant and the extract therefrom were assayed for CSF activity at each dilution ratio.

The results are given in Table 1 below, which also shows the results achieved by the same procedure as above using pcDE as a control in place of pcDM•CSF. The assay was conducted in duplicate.

Table 1

| Dilution ratio | 16 | 32 | 64 | 128 |
|---|---|---|---|---|
| pcDM·CSF | | | | |
| Supernatant | 282 | 274 | 159 | 79 |
| Extract | 97 | 41 | 0 | 0 |
| | | | | |
| pcDE (Control) | | | | |
| Supernatant | 0 | 0 | 0 | 0 |
| Extract | 0 | 0 | 0 | 0 |

Each value listed in Table 1 is the number of colonies per plate, the same as in the tables to follow showing the CSF activity.

(4) Preparation of M-CSF expression plasmid pcDM·CSF-185

Using the plasmid pcDM·CSF obtained by the procedure (2), the desired M-CSF expression plasmid pcDM·CSF-185 was prepared according to the site-specific mutagenesis method (Proc. Natl. Acad. Sci., 81, 5662-5666 (1984)) by replacing the codon (AAG) coding for the 186th amino acid (Lys) in Fig. 3 by an termination codon (TAG), the expression plasmid therefore having the 185th amino acid (Thr) in Fig. 3 at its C terminus.

This procedure will be described in detail below.

EcoRI-EcoRI DNA fragment (1.8 kb in size) was excised from plasmid pcDM·CSF and cloned in M13mp11 phage (RF) et EcoRI sites to thereby obtain a single-stranded (ss) DNA (M13-CSF), which was used as a template for mutagenesis.

On the other hand, synthetic oligonucleotide [5'-GTGGTGACCTAGCCTGATT-3' (primer)] was phosphorylated with T4 polynucleotide kinase and then hybridized with the ssDNA (M13-CSF). After annealing, the hybrid was treated with DNA polymerase I (Klenow fragment) and T4 DNA ligase in the presence of dNTP and incubated at 15°C for 18 hours.

The resulting DNA was introduced into JM105 competent cells, and 50 plaques among the plaques formed were inoculated on agar plate and incubated at 37°C for 18 hours. A filter containing the colonies was alkaline-denatured in a usual manner, dried and thereafter baked at 80°C for 2 hours. The filter was prehybridized and thereafter hybridized with $^{32}$P-probe prepared by labeling the 5' end of the above primer [$\gamma$-$^{32}$P]ATP at room temperature. The hybridized filter was washed with 6 x ssc (saline sodium citrate) first at room temperature for 10 minutes and then at 56°C for 4 minutes, dried and thereafter subjected to autoradiography at -70°C for 18 hours.

M13-CSF-185 was selected from among the mutant clones and transfected to JM105 to prepare ssDNA and RF DNA.

The exactness of the mutated sequence was confirmed by sequencing the ssDNA by the dideoxynucleotide chain termination method.

The desired plasmid pcDM·CSF-185 was obtained by preparing EcoRI-EcoRI fragment from the RF DNA amplified in the JM105 and inserting the fragment into an expression plasmid in the same manner as in the procedure (3).

Using this plasmid, COS-1 cells were caused to express r-MCSF in the same manner as in the procedure (3). Table 2 below shows the results.

Table 2

| Dilution ratio | 1 | 3 | 9 | 27 | 81 |
|---|---|---|---|---|---|
| pcDM•CSF-185 Supernatant | 288 | 243 | 238 | 173 | 90 |

(5) Preparation of M-CSF expression plasmid pcDM•CSF-177

Using 5'-GCTGAATGATCCAGCCAA-3' as a primer, the desired M-CSF expression plasmid pcDM•CSF-177 was prepared in the same manner as in the procedure (4) by replacing the codon (TGC) coding for the 178th amino acid (Cys) in Fig. 3 by a termination codon (TGA), the expression plasmid therefore having the 177th amino acid (Glu) in Fig. 3 at its C terminus.

Using this plasmid, COS-1 cells were caused to express r-MCSF in the same manner as in the procedure (3). Table 3 below shows the results.

Table 3

| Dilution ratio | 1 | 3 | 9 | 27 | 81 |
|---|---|---|---|---|---|
| pcDM•CSF-177 Supernatant | 249 | 254 | 166 | 83 | 28 |

The results given in Tables 2 and 3 reveal that the desired r-MCSF having M-CSF activity can be expressed by the use of plasmids pcDM•CSF-185 and pcDM•CSF-177 having the gene of the invention.

This indicates that the portion of the amino acid sequence shown in Fig. 3 from the 178th amino acid to the amino acid at the C terminus exerts substantially no influence on the expression of M-CSF molecules having the desired biological activity.

(6) Plasmid pcDM•CSF11 was obtained by the procedure (2) using λcM11cDNA in place of λcM5cDNA. r-MCSF was prepared by the procedure (3) with use of the plasmid, achieving substantially the same results as previously attained.

More specifically stated, λcM11 DNA was partially digested with restriction enzyme EcoRI, subjected to agarose gel electrophoresis, and a partially digested EcoRI fragment (about 2.5 kb) was purified.

The COS cell expression vector pcDE obtained by the procedure (1) was cleaved with restriction enzyme EcoRI and ligated to the cDNA fragment prepared above, using T4 DNA ligase, whereby the desired plasmid pcDM•CSF11 was obtained.

The plasmid thus obtained was introduced into E. Coli HB101 strain by transformation. The desired transformant was selected by the restriction enzyme analysis of it's plasmid DNA according to the alkaline lysis method (T. Maniatis et al., Molecular Cloning, pp 90, Cold Spring Harvor Laboratory (1982)-).

Further the cDNA (fragment partially digested with EcoRI) of the λcM11 DNA was inserted into cloning vector pUC19 DNA (product of Takara Shuzo Co., Ltd.) at EcoRI cloning site, giving plasmid pUC MCSF•11. The transformant obtained by introducing the plasmid into E. coli HB101 strain has been deposited under the name of Escherichia coli HB101/pUC MCSF•11 with deposition number FERM BP-1409 in Fermentation Research Institute, Agency of Industrial Science & Technology since July 16, 1987.

(7) Plasmid pcDM CSF11 was digested with restriction enzymes EcoRI and BstEII to isolate and purify EcoRI-BstEII DNA fragment of about 670 bp.

To the BstEII end of the DNA fragment was ligated a synthetic linker,

5'-GTGACCTGATAAGGATCCG-3'

3'-GACTATTCCTAGGCTTAA-5'

The resultant DNA fragment was then ligated to the above-mentioned plamid pcDE digested with EcoRI, using T4 DNA ligase to obtain the desired plasmid pcDM•CSF11-185. This plasmid has translation termination codon TGATAA at the site of the synthetic linker and therefore codes for a polypeptide having the 185th amino acid (Thr) in Fig. 5 at its C terminus.

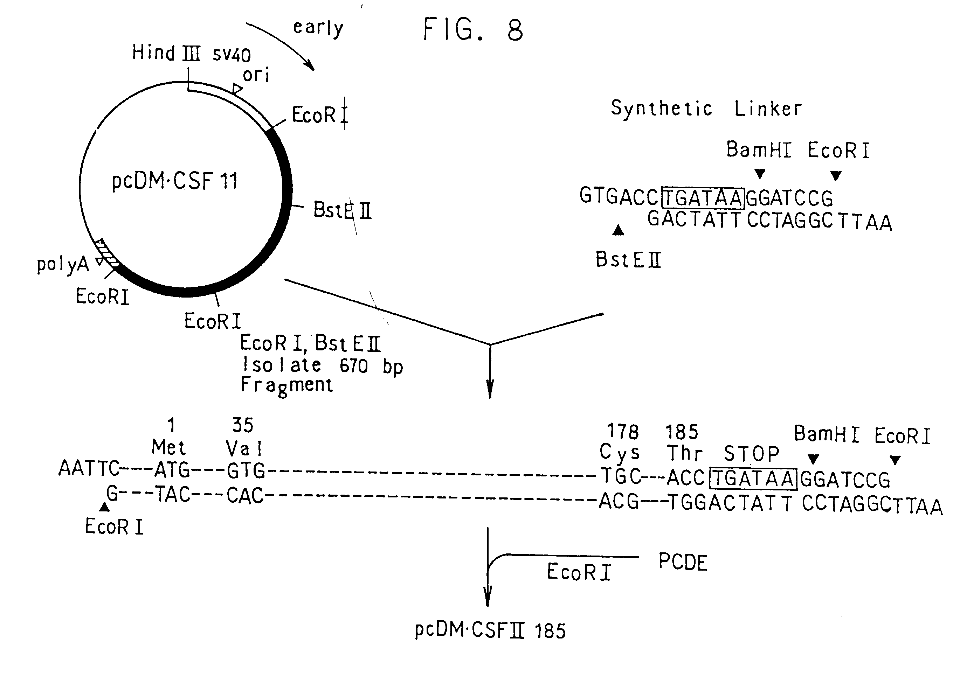

Fig. 8 schematically shows the above process.

15

Table 4 shows the result achieved in the same manner as in the procedure (3) using the plasmid.

Table 4

| Dilution ratio | 1 | 2 | 4 | 8 |
|---|---|---|---|---|
| pcDM·CSF11-185 Supernatant | >200 | >200 | 191 | 171 |

| Dilution ratio | 16 | 32 | 64 |
|---|---|---|---|
| pcDM·CSF11-185 Supernatant | 110 | 84 | 60 |

(8) Using the plasmid pcDM·CSF11-185 obtained by the procedure (7), the desired M-CSF expression plasmid pcDM·CSF11-177 was prepared by replacing the codon (TGC) coding for the 178th amino acid (Cys) in Fig. 5 by a termination codon (TGA) according to the procedure (5), the expression plasmid thus having the 177th amino acid (Glu) in Fig. 5 at its C terminus. The plasmid was used for expression in the same manner as in the procedure (3), with the result shown in Table 5 below.

Table 5

| Dilution ratio | 1 | 2 | 4 | 8 | 16 |
|---|---|---|---|---|---|
| pcDM·CSF11-177 Supernatant | 192 | >200 | >200 | 180 | 70 |

(9) Plasmid pcDM·CSF11-185 DNA was digested with EcoRI and BamHI to isolate and purify EcoRI-BamHI DNA fragment (about 680 bp), which was inserted into the EcoRI-BamHI cloning site of secretory expression vector pIN-III-OmpA3 (EMBO J., 3, 2437-2442(1984)) to obtain plasmid pIN-III-OmpA3-MCSF11-185.

The plasmid DNA was digested with XbaI and BamHI to isolate and purify XbaI-BamHI DNA fragment (about 770 bp), which was then inserted into the XbaI-BamHI cloning site of cloning vector pUC118 DNA (product of Takara Shuzo Co., Ltd.) for preparing a single-stranded DNA, whereby plasmid pUC118-OmpA3-MCSF11-185 was obtained.

Using the plasmid, plasmid pUC118-OmpA-MCSF11-NV151 was obtained by such modification in which the codon (GTG) coding for the 35th amino acid (Val) of the polypeptide shown in Fig. 5 can follow the codon (GCC) coding for the C-terminal amino acid (Ala) of OmpA signal peptide, according to the aforementioned method of site-specific mutagenesis.

Stated more specifically, E. coli JM105 transformed by plasmid pUC118-OmpA3-MCSF11-185 DNA was infected with helper phage K07 (product of Takara Shuzo Co., Ltd.), followed by incubation at 37° C for 14 hours, to obtain single-stranded (ss) pUC118-OmpA3-MCSF11-185DNA. With use of this DNA as a template for mutagenesis and also using 5'-TACCGTAGCGCAGGCCGTGTCGGAGTACTGTAGC-3' as a primer, the desired plasmid pUC118-OmpA-MCSF11-NV151 was obtained in the same manner as in the procedure (4).

The plasmid DNA was then digested with XbaI and BamHI to isolate and purify XbaI-BamHI DNA fragment (about 540 bp). The DNA fragment thus obtained was inserted into the XbaI-BamHI site of expression vector pIN-III(lpp^{P}-5)-A3, whereby the desired secretory expression plasmid pIN-III(lpp^{P}-5)-OmpA-MCSF11-NV151 was prepared for expressing a polypeptide comprising the portion of the sequence shown in Fig. 5 from the 35th amino acid (Val) to the 185th amino acid (Thr).

16

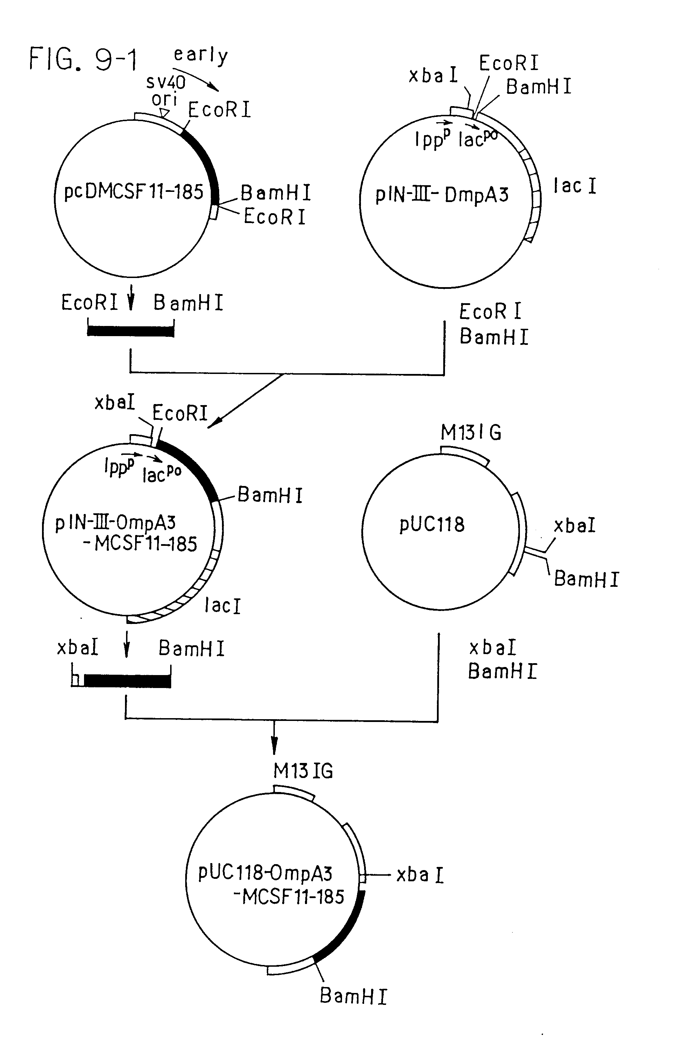

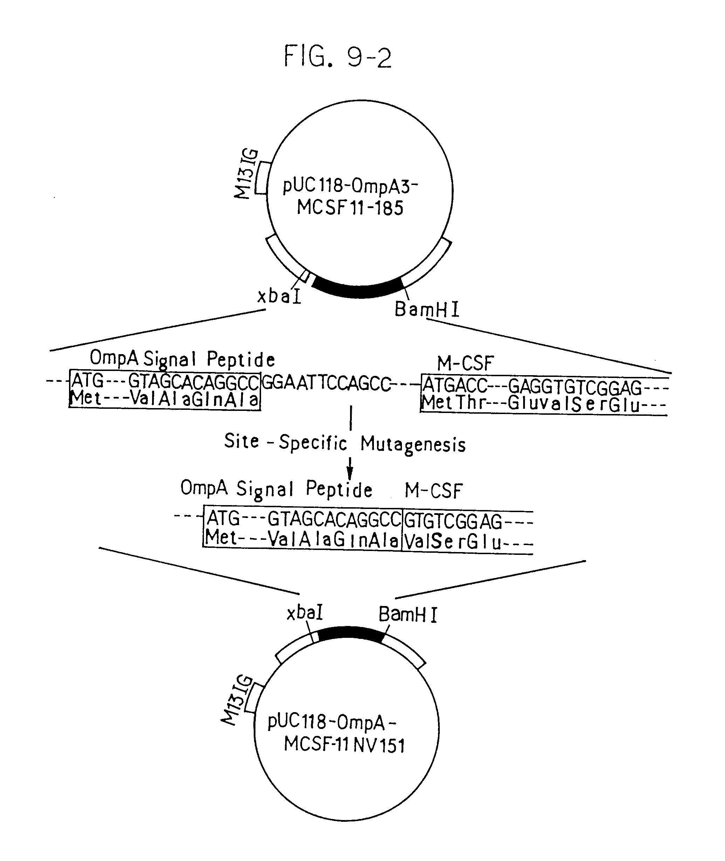

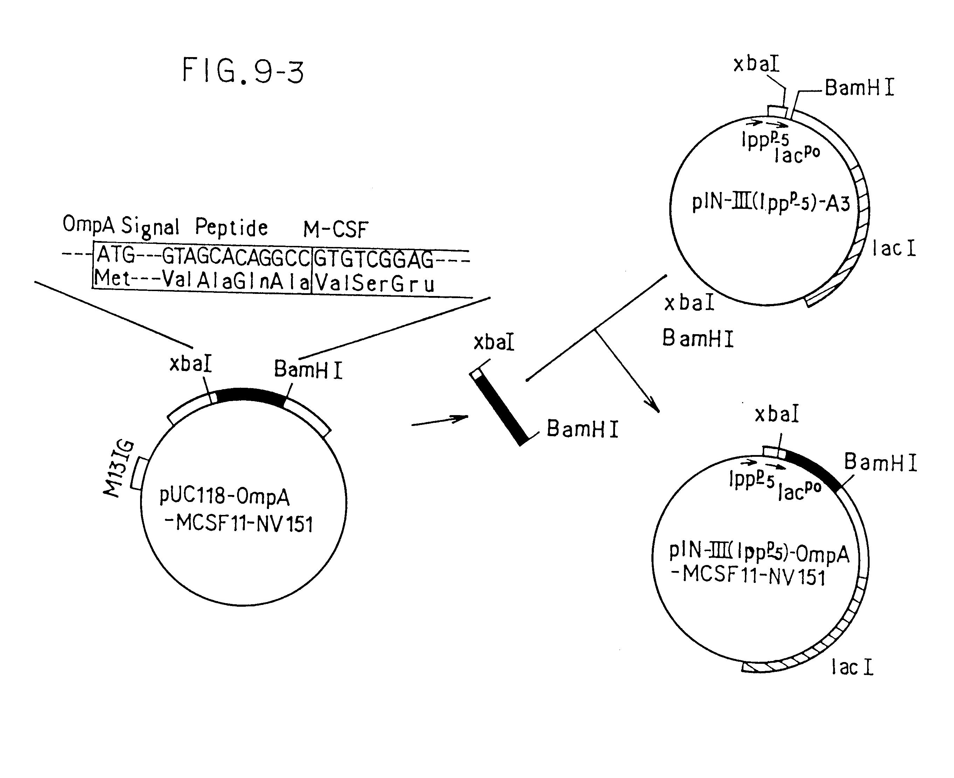

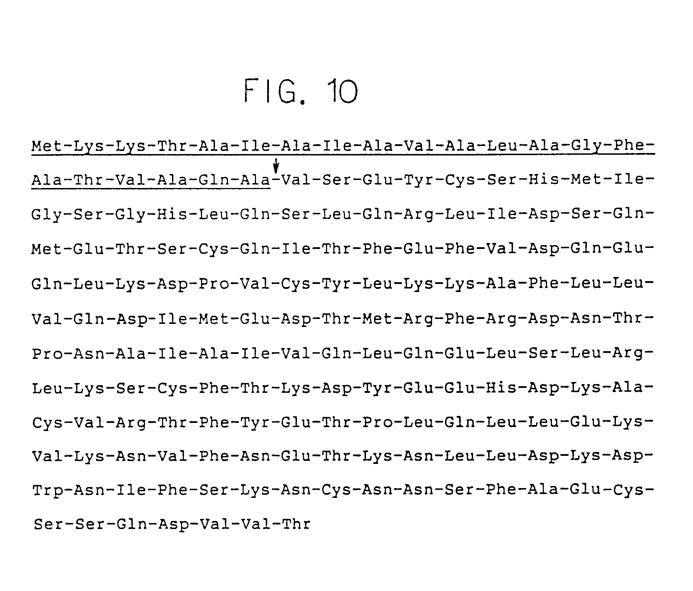

Figs. 9-1 to 9-3 schematically show the above process. Fig. 10 shows the amino acid sequence encoded in the secretory expression plasmid. In Fig. 10, the underlined portion of the sequence represents the OmpA signal peptide, while the processing site is marked with ↓.

(10) The pIN-III(lpp$^p$-5)-OmpA-MCSF11-NV151 obtained by the procedure (9) was introduced into E. coli HB101 and JM109 for the secretory production of the polypeptide into the periplasm and the periplasmic fraction having CSF activity was collected.

More specifically stated, the strain harboring the M-CSF secretory expression vector was incubated with shaking in 500 ml of LB medium containing 50 $\mu$g/ml of ampicillin at 37° C for 14 hours within a 2-liter flask, followed by centrifugation at room temperature at 5000 r.p.m. for 5 minutes. The cell pellets were suspended in 50 ml of 50 mM Tris-HCl (pH 8.0)-25% sucrose solution, and after addition of 1.25 ml of 250 mM EDTA (pH 8.0), the suspension was maintained at room temperature for 30 minutes with gentle shaking. The suspension was then centrifuged to obtain cell pellets, which were suspended again in 25 ml of ice-cold distilled water. The suspension was cooled in ice for 30 minutes with intermittent gentle shaking and thereafter centrifuged at 4°C at 10000 r.p.m. for 5 minutes to collect a supernatant as the periplasmic fraction.

(11) The desired M-CSF secretory expression plasmid, pIN-III(lpp$^p$-5)-OmpA-MCSF11-NV143, was obtained for expressing a polypeptide comprising the portion of the sequence shown in Fig. 5 from the 35th amino acid (Val) to the 177th amino acid (Glu), by the procedure (5) using pcDM•CSF11-177 in place of pcDM•CSF11-185.

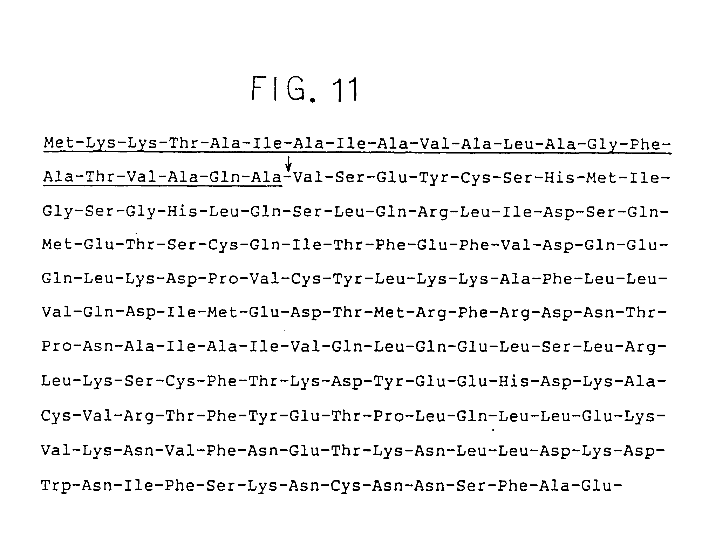

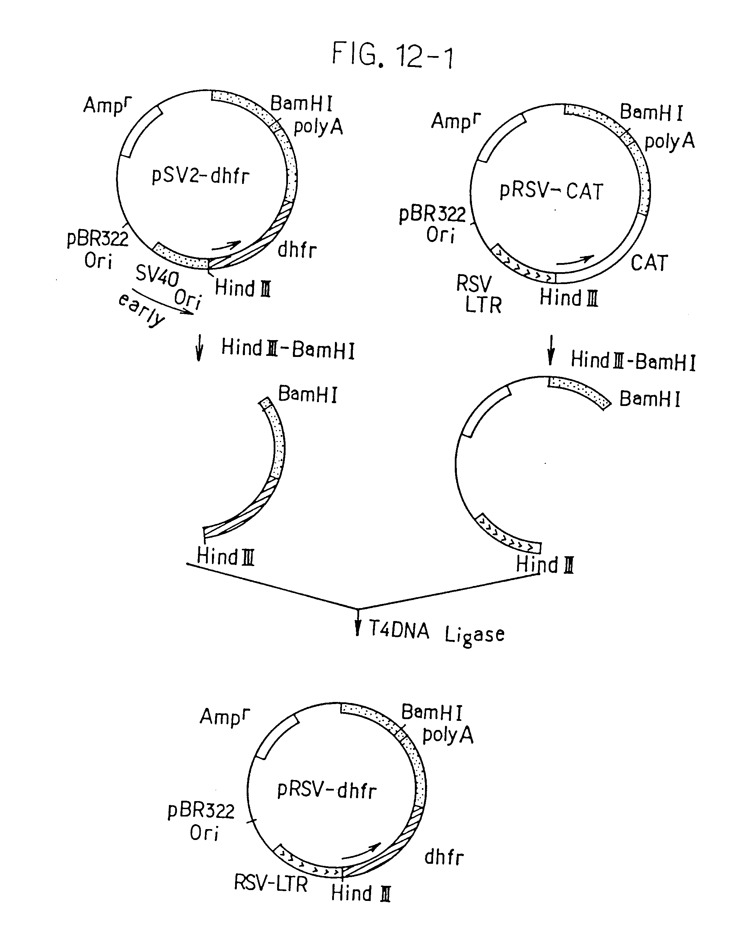

Fig. 11 shows the amino acid sequence encoded in the plasmid. In Fig. 11, the underlined portion of the sequence represents OmpA signal peptide, and the mark ↓ indicates the processing site. In the same manner as in the procesure (10), the plasmid was introduced into E. coli, from which a supernatant exhibiting CSF activity was obtained as a periplasmic fraction.

(12) The supernatants obtained by the procedures (6), (7), (8) and (10) were subjected to HPLC under the following conditions.

Column:            TSK Gel G3000SW (60 cm x 7.5 mm(diam.), Toyo Soda Mfg. Co., Ltd.
Eluent:            PBS-containing 0.005% polyethylene glycol and 0.15 M NaCl.
Flow rate:         0.8 ml/min.
Fraction volume:   0.8 ml/tube/min.

The following results were obtained for the samples with reference to molecular weight markers (glutamate dehydrogenase: 290,000 lactate dehydrogenase: 142,000, enolase: 67,000, adenylate kinase: 32,000, cytochrome C: 12,400).

Sample:    Four ml of the culture supernatant obtained by the procedure (6) was concentrated to about 150 $\mu$l by Centricon-10 (product of Amicon) CSF activity was found in a fraction corresponding to 320,000 to 700,000 in molecular weight range (480,000 on the average).

Sample:    Four ml of the culture supernatant obtained by the procedure (7) was treated in the same manner as above. CSF activity was found in a fraction corresponding to 66,000 to 86,000 in molecular weight range (76,000 on the average).

Sample:    Four ml of the culture supernatant obtained by  the procedure (8) was treated in the same manner as above. CSF activity was found in a fraction corresponding to 52,000 to 86,000 in molecular weight range (67,000 on the average).

Sample:    Two ml of the supernatant of periplasmic fraction obtained by the procedure (10) was concentrated to about 110 $\mu$l in the same manner as above. CSF activity was found at a position corresponding to about 30,000 to 40,000 in molecular weight range (35,000 on the average).

(13) Ten ml of the culture supernatant obtained by the procedure (6), (7) or (8) was passed through a column packed with 1 ml of ConA-Sepharose, which was then washed with 5 ml of PBS⁻ and eluted with PBS⁻ (10 ml) containing 0.5 M methyl-$\alpha$-D-mannoside. A 4-ml portion of the resulting eluate was concentrated with Centricon-10, and the concentrate was used for SDS-PAGE mentioned below.

The periplasmic supernatant as obtained by the procedure (10) was used as another sample therefor. M-CSF was detected by using a rabbit anti-serum against M-CSF prepared in the conventional manner, after Western blotting of the SDS-PAGE gels.

More specifically, SDS-PAGE was carried out according to the method of Laemmli, U. K. (Nature, 277, 680(1979)) using a mini-vertical slab cell (gel concentration 15%). Western blotting was conducted using Transblot Cell (product of Bio-Rad Laboratories). The nitrocellulose filter having the desired substance transferred thereto, was blocked with PBS⁻ containing 1% bovine serum albumin and then reacted with the rabbit anti-serum against M-CSF first and further with peroxidase-labeled goat anti-rabbit antibody (product of Bio-Rad Laboratories). The resulting nitrocellulose filter was reacted with 4-chloro-1-

EP 0 547 026 A1

naphthol solution serving as a color developing substrate for the detection of M-CSF band. Table 6 shows the results.

## Table 6

| Sample | Molecular weight of detectd band (kd) | |
|---|---|---|
| | Non-reducing condition | Reducing condition |
| Supernatant Obtained by (6) | 82 | 43.5 |
| | 150 | 58 |
| | At least 200 (in stacking gel) | At least 70 (plural) |
| Supernatant obtained by (7) | 40 (faint) | 15 (faint) |
| | 43 | 20.5 |
| | 45 | 26 |
| | 47 | |
| Supernatant obtained by (8) | 44 | 19 (faint) |
| | 46 | 25 |
| | 50 | |
| Supernatant obtained by (10) | 32 | 17 |

(14) Plasmid pSV2-dhfr (Mol. Cell. Biol., 1, 854 (1981)) was cleaved into two fragments with restriction enzymes HindIII and BamHI, and the fragment containing a dihydrofolate reductase (DHFR) gene was isolated and purified.

Next, plasmid pRSV-CAT (Proc. Natl. Acad. Sci. U.S.A., 79, 6777(1981)) was similarly cleaved with HindIII and BamHI, and the fragment containing the LTR (long terminal repeat) portion of Rous sarcoma virus (RSV) was isolated and purified. The two purified fragments were ligated together using T4 DNA ligase.

E. coli HB101 competent cells were transformed by adding the above ligated product. Plasmid DNAs were prepared from ampicillin-resistant colonies and were digested with restriction enzymes. The desired plasmid pRSV-dhfr which showed the predicted restriction enzyme map was obtained.

This plasmid contains the LTR portion of RSV, DHFR gene, intervening sequence derived from SV40, polyadenylation signal, and further a replication origin and ampicillin-resistant gene derived from E. coli plasmid pBR322, and expresses DHFR under the control of a promotor contained in the LTR portion.

The plasmid pRSV-dhfr was cleaved into two fragments with NdeI and BamHI, and the fragment containing the DHFR gene was isolated and purified. The DNA fragment obtained was treated with DNA polymerase I (Klenow fragment) to convert the cohesive ends to blunt ends.

On the other hand, the plasmid pcDM•CSF11 obtained by the procedure (6) was cleaved with SalI, and the cleaved ends were similarly converted to the blunt ends by treatment with DNA polymerase I.

The two fragments obtained were ligated together with T4 DNA ligase, and the ligated product was transformed to E. coli JM-109 competent cells. Plasmid DNAs were prepared from the ampicillin-resistant colonies obtained, restriction enzyme maps prepared, and the desired pcDM•CSF11-dhfr for expressing both M-CSF and DHFR genes was obtained.

18

This plasmid contains the following sequences: with respect to M-CSF gene expression, SV40 early promoter, SV40-derived intervening sequence, M-CSF gene and polyadenylation signal, and also with respect to DHFR gene expression, RSV-derived LTR, DHFR gene, SV40 early promoter and polyadenylation signal. The plasmid further contains a replication origin and ampicillin-resistant gene derived from E. coli plasmid pBR322.

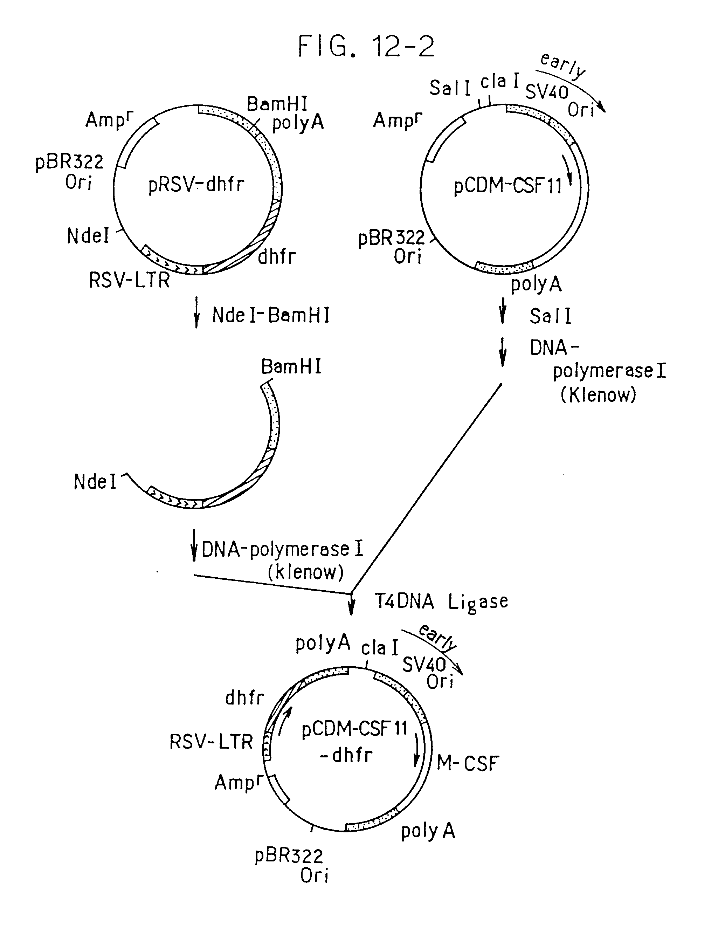

Figs. 12-1 and 12-2 schematically show the above process.

(15) Using the plasmid pcDM CSF11-dhfr thus obtained, r-MCSF was produced by the procedure (3). The CSF activity of the culture supernatant obtained was 68 in terms of the number of colonies (average) per plate (0 for plasmid pcDE as a control).

(16) Chinese hamster ovary dhfr-deficient cells (CHO-DuK dhfr⁻ cells; Proc. Natl. Acad. Sci. U.S.A., 77, 4216(1980)) incubated in 75-cm$^2$ incubator flask (product of Costar Corp.) were treated with trypsin (product of Flow Lab. Inc.) in the usual manner, suspended in Dulbecco's modified minimum essential medium (DMEM, product of GIBCO Laboratories Life technologies Inc.) supplemented with 10% dialyzed FCS (product of GIBCO), washed with the same medium and separated from the medium.

The cells were then suspended in a DNA injection solution (0.25 M mannitol-0.1 mM CaCl$_2$•2H$_2$O-0.1 mM MgSO$_4$•7H$_2$O-0.2 mM Tris-HCl (pH 7.2), Wako Pure Chemical Industries Ltd.).

The M-CSF expression plasmid pcDM•CSF11-dhfr obtained by the procedure (14) was cleaved into linear form with ClaI and thereafter dissolved in the DNA injection solution.

The cell suspension (200 μl, 2 x 10$^6$ cells) and the DNA solution (200 μl, 30 μg of DNA) were mixed together, and the mixture was placed into Fusion Chamber CH-2 (product of Shimadzu Seisakusho Ltd.), which was connected to Electric Fusion Device SSH-1 (product of Shimnadzu Seisakusho Ltd.). 4.2 kV/cm$^2$ pulse was applied to the mixture twice at an interval of 1 second to electrically transfect the DNA into the cells.

The cells having the DNA transfected therein were suspended in 10% dialyzed FCS-1% nonessential amino acid solution (product of Flow Lab. Inc.)-2% HT solution (product of Flow Lab. Inc.)-DMEM medium and incubated on a 24-well plate (product of Costar Corp.).

After incubation for 48 hours, the medium was replaced by a selective medium (DMEM medium containing 10% dialyzed FCS and 1% nonessential amino acid solution). The medium was thereafter replaced by a fresh one every 3 to 4 days.

From among about 200 clones of transformant cells obtained by incubating for 10 to 14 days, 50 clones were selected, treated with trypsin in the usual manner and transferred to a new 24-well plate.

The amplification of the DHFR gene gives resistance to methotrexate (MTX) of high concentration (J.B.C., 253, 1357(1978)), while the gene cotransfected with a DHFR gene are amplified by MTX (J. Mol. Biol., 159, 601 (1982)).

The 50 clones of transformant cells were further incubated in a selective medium containing 20 nM MTX. The proliferated resistant clones were treated with trypsin, then suspended in a selective medium containing 50 nM MTX and incubated. Similarly, the proliferated resistant clones were further incubated in a selective medium containing 100 nM MTX, eventually affording clones which were resistant to 400 nM MTX.

Table 7 below shows the CSF activity of the supernatants of cultures of these resistant clones.

Table 7

| CHO cells Clone No. | Resistant to: | |
|---|---|---|
| | MTX 100nM | MTX 400nM |
| 2,3-8 | 2940 | 30240 |
| 2,3-15 | 2480 | 13770 |
| 2,3-18 | 6060 | 22770 |
| 2,3-20 | 4380 | 24030 |
| 2,3-42 | 3900 | 19530 |

The culture supernatant of CHO cells as prepared by the above procedure was directly used as a sample for SDS-PAGE and analyzed for molecular weight in the same manner as in the procedure (13). Consequently, the M-CSF band was detected at a position corresponding to 90,000 under a non-reducing condition and to 44,000 under a reducing condition.

**Claims**

1.  A DNA molecule encoding a biologically active human M-CSF having a nucleotide molecule coding for an amino acid sequence having any one of amino acids from 33rd amino acid Glu to 37th amino acid Glu at its N-terminus and any one of amino acids from 177th amino acid Glu to 185th amino acid Thr at its C-terminus of the sequence as shown in formula (1) as follows:

```
      1                                    10
    Met-Thr-Ala-Pro-Gly-Ala-Ala-Gly-Arg-Cys-
                                         20
    Pro-Pro-Thr-Thr-Trp-Leu-Gly-Ser-Leu-Leu-
                                         30
    Leu-Leu-Val-Cys-Leu-Leu-Ala-Ser-Arg-Ser-
                                         40
    Ile-Thr-Glu-Glu-Val-Ser-Glu-Tyr-Cys-Ser-
                                         50
    His-Met-Ile-Gly-Ser-Gly-His-Leu-Gln-Ser-
                                         60
    Leu-Gln-Arg-Leu-Ile-Asp-Ser-Gln-Met-Glu-
                                         70
    Thr-Ser-Cys-Gln-Ile-Thr-Phe-Glu-Phe-Val-
                                         80
    Asp-Gln-Glu-Gln-Leu-Lys-Asp-Pro-Val-Cys-
                                         90
    Tyr-Leu-Lys-Lys-Ala-Phe-Leu-Leu-Val-Gln-
                                        100
     X -Ile-Met-Glu-Asp-Thr-Met-Arg-Phe-Arg-
                                        110
    Asp-Asn-Thr-Pro-Asn-Ala-Ile-Ala-Ile-Val-
                                        120
    Gln-Leu-Gln-Glu-Leu-Ser-Leu-Arg-Leu-Lys-
                                        130
    Ser-Cys-Phe-Thr-Lys-Asp-Tyr-Glu-Glu-His-
                                        140
    Asp-Lys-Ala-Cys-Val-Arg-Thr-Phe-Tyr-Glu-
                                        150
    Thr-Pro-Leu-Gln-Leu-Leu-Glu-Lys-Val-Lys-
                                        160
    Asn-Val-Phe-Asn-Glu-Thr-Lys-Asn-Leu-Leu-
```

```
                                        170
Asp-Lys-Asp-Trp-Asn-Ile-Phe-Ser-Lys-Asn-
                                        180
Cys-Asn-Asn-Ser-Phe-Ala-Glu-Cys-Ser-Ser-
                                        190
Gln-Asp-Val-Val-Thr-Lys-Pro-Asp-Cys-Asn-
                                        200
Cys-Leu-Tyr-Pro-Lys-Ala-Ile-Pro-Ser-Ser-
                                        210
Asp-Pro-Ala-Ser-Val-Ser-Pro-His-Gln-Pro-
                                        220
Leu-Ala-Pro-Ser-Met-Ala-Pro-Val-Ala-Gly-
                                        230
Leu-Thr-Trp-Glu-Asp-Ser-Glu-Gly-Thr-Glu-
                                        240
Gly-Ser-Ser-Leu-Leu-Pro-Gly-Glu-Gln-Pro-
                                        250
Leu-His-Thr-Val-Asp-Pro-Gly-Ser-Ala-Lys-
                                        260
Gln-Arg-Pro-Pro-Arg-Ser-Thr-Cys-Gln-Ser-
                                        270
Phe-Glu-Pro-Pro-Glu-Thr-Pro-Val-Val-Lys-
                                        280
Asp-Ser-Thr-Ile-Gly-Gly-Ser-Pro-Gln-Pro-
                                        290
Arg-Pro-Ser-Val-Gly-Ala-Phe-Asn-Pro-Gly-
                                        300
Met-Glu-Asp-Ile-Leu-Asp-Ser-Ala-Met-Gly-
                                        310
Thr-Asn-Trp-Val-Pro-Glu-Glu-Ala-Ser-Gly-
                                        320
Glu-Ala-Ser-Glu-Ile-Pro-Val-Pro-Gln-Gly-
                                        330
Thr-Glu-Leu-Ser-Pro-Ser-Arg-Pro-Gly-Gly-
                                        340
Gly-Ser-Met-Gln-Thr-Glu-Pro-Ala-Arg-Pro-
                                        350
Ser-Asn-Phe-Leu-Ser-Ala-Ser-Ser-Pro-Leu-
                                        360
Pro-Ala-Ser-Ala-Lys-Gly-Gln-Gln-Pro-Ala-
                                        370
Asp-Val-Thr-Gly-Thr-Ala-Leu-Pro-Arg-Val-
                                        380
Gly-Pro-Val-Arg-Pro-Thr-Gly-Gln-Asp-Trp-
                                        390
Asn-His-Thr-Pro-Gln-Lys-Thr-Asp-His-Pro-
                                        400
Ser-Ala-Leu-Leu-Arg-Asp-Pro-Pro-Glu-Pro-
                                        410
Gly-Ser-Pro-Arg-Ile-Ser-Ser-Pro-Arg-Pro-
```

```
                                           420
          Gln-Gly-Leu-Ser-Asn-Pro-Ser-Thr-Leu-Ser-
                                           430
          Ala-Gln-Pro-Gln-Leu-Ser-Arg-Ser-His-Ser-
                                           440
          Ser-Gly-Ser-Val-Leu-Pro-Leu-Gly-Glu-Leu-
                                           450
          Glu-Gly-Arg-Arg-Ser-Thr-Arg-Asp-Arg-Arg-
                                           460
          Ser-Pro-Ala-Glu-Pro-Glu-Gly-Gly-Pro-Ala-
                                           470
          Ser-Glu-Gly-Ala-Ala-Arg-Pro-Leu-Pro-Arg-
                                           480
          Phe-Asn-Ser-Val-Pro-Leu-Thr-Asp-Thr-Gly-
                                           490
          His-Glu-Arg-Gln-Ser-Glu-Gly-Ser-Ser-Ser-
                                           500
          Pro-Gln-Leu-Gln-Glu-Ser-Val-Phe-His-Leu-
                                           510
          Leu-Val-Pro-Ser-Val-Ile-Leu-Val-Leu-Leu-
                                           520
          Ala-Val-Gly-Gly-Leu-Leu-Phe-Tyr-Arg-Trp-
                                           530
          Arg-Arg-Arg-Ser-His-Gln-Glu-Pro-Gln-Arg-
                                           540
          Ala-Asp-Ser-Pro-Leu-Glu-Gln-Pro-Glu-Gly-
                                           550
          Ser-Pro-Leu-Thr-Gln-Asp-Asp-Arg-Gln-Val-
                                           554
          Glu-Leu-Pro-Val
```

wherein X is Tyr or Asp.

2. A DNA molecule as defined in Claim 1 which has a nucleotide molecule coding for the amino acid sequence from 35th amino acid Val to 185th amino acid Thr wherein X represents Asp.

3. A DNA molecule as defined in Claim 1 which has a nucleotide molecule coding for the amino acid sequence from 36th amino acid Ser to 185th amino acid Thr wherein X represents Asp.

4. A DNA molecule as defined in Claim 1 which has a nucleotide molecule coding for the amino acid sequence shown in Figure 10 and Figure 11.

5. An expression vector including the DNA molecule defined in Claim 1 and capable of expressing a biologically active human M-CSF.

6. A transformant harbouring the expression vector defined in Claim 5 and capable of producing a biologically active human M-CSF.

7. A process for preparing a recombinant human M-CSF, which comprises culturing a transformant harbouring an expression vector capable of expressing a biologically active human M-CSF and collecting the M-CSF thus produced, said expression vector including the DNA molecule defined in any one of Claims 1 to 4.

8. A process as defined in Claim 7, wherein said transformant is a procaryote.

22

FIG. 1

# FIG. 2-1

5' AGCCGCTCTC CGCATCCCAG GACAGCGGTG CGGCCCTCGG CCGGGGCGCC
TCGGCGAGAG GCGTAGGGTC CTGTCGCCAC GCCGGGAGCC GGCCCCGCGG

CACTCCGCAG CACCCAGCGA GCGAGCGAGC GAGCGAGGGC GGCCGACGCG
GTGAGGCGTC GTGGGTCGCT CGCTCGCTCG CTCGCTCCCG CCGGCTGCGC

CCCGGCCGGG ACCCAGCTGC CCGTATGACC GCGCCGGGCG CCGCCGGGCG
GGGCCGGCCC TGGGTCGACG GGCATACTGG CGCGGCCCGC GGCGGCCCGC

CTGCCCTCCC ACGACATGGC TGGGCTCCCT GCTGTTGTTG GTCTGTCTCC
GACGGGAGGG TGCTGTACCG ACCCGAGGGA CGACAACAAC CAGACAGAGG

TGGCGAGCAG GAGTATCACC GAGGAGGTGT CGGAGTACTG TAGCCACATG
ACCGCTCGTC CTCATAGTGG CTCCTCCACA GCCTCATGAC ATCGGTGTAC

ATTGGGAGTG GACACCTGCA GTCTCTGCAG CGGCTGATTG ACAGTCAGAT
TAACCCTCAC CTGTGGACGT CAGAGACGTC GCCGACTAAC TGTCAGTCTA

GGAGACCTCG TGCCAAATTA CATTTGAGTT TGTAGACCAG GAACAGTTGA
CCTCTGGAGC ACGGTTTAAT GTAAACTCAA ACATCTGGTC CTTGTCAACT

AAGATCCAGT GTGCTACCTT AAGAAGGCAT TTCTCCTGGT ACAATACATA
TTCTAGGTCA CACGATGGAA TTCTTCCGTA AAGAGGACCA TGTTATGTAT

ATGGAGGACA CCATGCGCTT CAGAGATAAC ACCCCCAATG CCATCGCCAT
TACCTCCTGT GGTACGCGAA GTCTCTATTG TGGGGGTTAC GGTAGCGGTA

TGTGCAGCTG CAGGAACTCT CTTTGAGGCT GAAGAGCTGC TTCACCAAGG
ACACGTCGAC GTCCTTGAGA GAAACTCCGA CTTCTCGACG AAGTGGTTCC

ATTATGAAGA GCATGACAAG GCCTGCGTCC GAACTTTCTA TGAGACACCT
TAATACTTCT CGTACTGTTC CGGACGCAGG CTTGAAAGAT ACTCTGTGGA

CTCCAGTTGC TGGAGAAGGT CAAGAATGTC TTTAATGAAA CAAAGAATCT
GAGGTCAACG ACCTCTTCCA GTTCTTACAG AAATTACTTT GTTTCTTAGA

CCTTGACAAG GACTGGAATA TTTTCAGCAA GAACTGCAAC AACAGCTTTG
GGAACTGTTC CTGACCTTAT AAAAGTCGTT CTTGACGTTG TTGTCGAAAC

CTGAATGCTC CAGCCAAGAT GTGGTGACCA AGCCTGATTG CAACTGCCTG
GACTTACGAG GTCGGTTCTA CACCACTGGT TCGGACTAAC GTTGACGGAC

TACCCCAAAG CCATCCCTAG CAGTGACCCG GCCTCTGTCT CCCCTCATCA
ATGGGGTTTC GGTAGGGATC GTCACTGGGC CGGAGACAGA GGGGAGTAGT

EP 0 547 026 A1

# FIG. 2-2

```
GCCCCTCGCC CCCTCCATGG CCCCTGTGGC TGGCTTGACC TGGGAGGACT
CGGGGAGCGG GGGAGGTACC GGGGACACCG ACCGAACTGG ACCCTCCTGA

CTGAGGGAAC TGAGGGCAGC TCCCTCTTGC CTGGTGAGCA GCCCCTGCAC
GACTCCCTTG ACTCCCGTCG AGGGAGAACG GACCACTCGT CGGGGACGTG

ACAGTGGATC CAGGCAGTGC CAAGCAGCGG CCACCCAGGA GCACCTGCCA
TGTCACCTAG·GTCCGTCACG GTTCGTCGCC GGTGGGTCCT CGTGGACGGT

GAGCTTTGAG CCGCCAGAGA CCCCAGTTGT CAAGGACAGC ACCATCGGTG
CTCGAAACTC GGCGGTCTCT GGGGTCAACA GTTCCTGTCG TGGTAGCCAC

GCTCACCACA GCCTCGCCCC TCTGTCGGGG CCTTCAACCC CGGGATGGAG
CGAGTGGTGT CGGAGCGGGG AGACAGCCCC GGAAGTTGGG GCCCTACCTC

GATATTCTTG ACTCTGCAAT GGGCACTAAT TGGGTCCCAG AAGAAGCCTC
CTATAAGAAC TGAGACGTTA CCCGTGATTA ACCCAGGGTC TTCTTCGGAG

TGGAGAGGCC AGTGAGATTC CCGTACCCCA AGGGACAGAG CTTTCCCCCT
ACCTCTCCGG TCACTCTAAG GGCATGGGGT TCCCTGTCTC GAAAGGGGGA

CCAGGCCAGG AGGGGGCAGC ATGCAGACAG AGCCCGCCAG ACCCAGCAAC
GGTCCGGTCC TCCCCCGTCG TACGTCTGTC TCGGGCGGTC TGGGTCGTTG

TTCCTCTCAG CATCTTCTCC ACTCCCTGCA TCAGCAAAGG|GCCAACAGCC
AAGGAGAGTC GTAGAAGAGG TGAGGACGT AGTCGTTTCC|CGGTTGTCGG

GGCAGATGTA ACTGGTACCG CCTTGCCCAG GGTGGGCCCG TGAGCATGGC
CCGTCTACAT TGACCATGGC GGAACGGGTC CCACCCGGGC ACTCGTACCG

CAGGACTGGA ATCACACCCC CCAGAAGAGA CACCATCCAT CTGCCCTGCT
GTCCTGACCT TAGTGTGGGG GGTCTTCTCT GTGGTAGGTA GACGGGACGA

CAGAGACCCC CCGGAGCCAG GCTCTCCCAG GATCTCATCA CCGCGCCCCC
GTCTCTGGGG GGCCTCGGTC CGAGAGGGTC CTAGAGTAGT GGCGCGGGGG

AGGGCCTCAG CAACCCCTCC ACCCTCTGCT GCTCAGCCAC AGCTTTCCAG
TCCCGGAGTC GTTGGGGAGG TGGGAGACGA CGAGTCGGTG TCGAAAGGTC

AAGCCACTCC TCGGGCGTGC TGCCCTTGGG GAGCTGGAGG GCAGGAGGAG
TTCGGTGAGG AGCCCGCACG ACGGGAACCC CTCGACCTCC CGTCCTCCTC

CACCAGGGAT CGGAGGAGCC CCGCAGAGCC AGAAGGAGGA CCAGCAAGTG
GTGGTCCCTA GCCTCCTCGG GGCGTCTCGG TCTTCCTCCT GGTCGTTCAC
```

25

# FIG. 2-3

```
AAGGCAGCCA GCCCCTGCCC CGTTTTAACT CCGTTCCTTT GACTGACACA
TTCCGTCGGT CGGGGACGGG GCAAAATTGA GGCAAGGAAA CTGACTGTGT

GGCCATGAGA GGCAGTCCGA GGGATCCTCC AGCCCGCAGC TCCAGGAGTC
CCGGTACTCT CCGTCAGGCT CCCTAGGAGG TCGGGCGTCG AGGTCCTCAG

TGTCTTCCAC CTGCTGGTGC CCAGTGTCAT CCTGGTCTTG CTGGCCGTCG
ACAGAAGGTG GACGACCACG GGTCACAGTA GGACCAGAAC GACCGGCAGC

GAGGCCTCTT GTTCTACAGG TGGAGGCGGC GGAGCCATCA AGAGCCTCAG
CTCCGGAGAA CAAGATGTCC ACCTCCGCCG CCTCGGTAGT TCTCGGAGTC

AGAGCGGATT CTCCCTTGGA GCAACCAGAG GGCAGCCCCC TCACTCAGGA
TCTCGCCTAA GAGGGAACCT CGTTGGTCTC CCGTCGGGGG AGTGAGTCCT

TGACAGACAG GTGGAACTGC CAGTGTAGAG GGAATTCTAA GACCCCTCAC
ACTGTCTGTC CACCTTGACG GTCACATCTC CCTTAAGATT CTGGGGAGTG

CATCCTGGAC ACTCTCGTTT GTCAATGTCC CTCTGAAAAT GTGACGCCCA
GTAGGACCTG TGAGAGCAAA CAGTTACAGG GAGACTTTTA CACTGCGGGT

GCCCCGGACA CAGTACTCCA GATGTTGTCT GACCAGCTCA GAGAGAGTAC
CGGGGCCTGT GTCATGAGGT CTACAACAGA CTGGTCGAGT CTCTCTCATG

AGTGGGACTG TTACCTTCCT TGATATGGAC AGTATTCTTC TATTTGTGCA
TCACCCTGAC AATGGAAGGA ACTATACCTG TCATAAGAAG ATAAACACGT

GATTAAGATT GCATTAGTTT TTTTCTTAAC AACTGCATCA TACTGTTGTC
CTAATTCTAA CGTAATCAAA AAAAGAATTG TTGACGTAGT ATGACAACAG

ATATGTTGAG CCTGTGGTCT ATAAAACCCC TAGTTCCATT TCCCATAAAC
TATACAACTC GGACACCAGA TATTTTGGGG ATCAAGGTAA AGGGTATTTG

TTCTGTCAAG CCAGACCATC TCTACCCTGT ACTTGGACAA CTTAACTTTT
AAGACAGTTC GGTCTGGTAG AGATGGGACA TGAACCTGTT GAATTGAAAA

TTAACCAAAG TGCAGTTTAT GTTCACCTTT GTTAAAGCCA CCTTGTGGTT
AATTGGTTTC ACGTCAAATA CAAGTGGAAA CAATTTCGGT GGAACACCAA

TCTGCCCATC ACCTGAACCT ACTGAAGTTG TGTGAAATCC TAATTCTGTC
AGACGGGTAG TGGACTTGGA TGACTTCAAC ACACTTTAGG ATTAAGACAG

ATCTCCGTAG CCCTCCCAGT TGTGCCTCCT GCACATTGAT GAGTGCCTGC
TAGAGGCATC GGGAGGGTCA ACACGGAGGA CGTGTAACTA CTCACGGACG
```

26

# FIG. 2-4

```
TGTTGTCTTT GCCCATGTTG TTGATGTAGC TGTGACCCTA TTGTTCCTCA
ACAACAGAAA CGGGTACAAC AACTACATCG ACACTGGGAT AACAAGGAGT

CCCCTGCCCC CCGCCAACCC CAGCTGGCCC ACCTCTTCCC CCTCCCACCC
GGGGACGGGG GGCGGTTGGG GTCGACCGGG TGGAGAAGGG GGAGGGTGGG

AAGCCCACAG CCAGCCCATC AGGAAGCCTT CCTGGCTTCT CCACAACCTT
TTCGGGTGTC GGTCGGGTAG TCCTTCGGAA GGACCGAAGA GGTGTTGGAA

CTGACTGTCT TTTCAGTCAT GCCCCCTGCT CTTTTGTATT TGGCTAATAG
GACTGACAGA AAAGTCAGTA CGGGGGACGA GAAAACATAA ACCGATTATC

TATATCAATT TGCACTTAAA AAAAAAAAAA AAAAAAAA 3'
ATATAGTTAA ACGTGAATTT TTTTTTTTTT TTTTTTTT
```

# FIG. 3-1

ATG.ACC.GCG.CCG.GGC.GCC.GCC.GGG.CGC.TGC.CCT.CCC.ACG.ACA.TGG.
Met-Thr-Ala-Pro-Gly-Ala-Ala-Gly-Arg-Cys-Pro-Pro-Thr-Thr-Trp-

CTG.GGC.TCC.CTG.CTG.TTG.TTG.GTC.TGT.CTC.CTG.GCG.AGC.AGG.AGT.
Leu-Gly-Ser-Leu-Leu-Leu-Leu-Val-Cys-Leu-Leu-Ala-Ser-Arg-Ser-

ATC.ACC.GAG.GAG.GTG.TCG.GAG.TAC.TGT.AGC.CAC.ATG.ATT.GGG.AGT.
Ile-Thr-Glu-Glu-Val-Ser-Glu-Tyr-Cys-Ser-His-Met-Ile-Gly-Ser-

GGA.CAC.CTG.CAG.TCT.CTG.CAG.CGG.CTG.ATT.GAC.AGT.CAG.ATG.GAG.
Gly-His-Leu-Gln-Ser-Leu-Gln-Arg-Leu-Ile-Asp-Ser-Gln-Met-Glu-

ACC.TCG.TGC.CAA.ATT.ACA.TTT.GAG.TTT.GTA.GAC.CAG.GAA.CAG.TTG.
Thr-Ser-Cys-Gln-Ile-Thr-Phe-Glu-Phe-Val-Asp-Gln-Glu-Gln-Leu-

AAA.GAT.CCA.GTG.TGC.TAC.CTT.AAG.AAG.GCA.TTT.CTC.CTG.GTA.CAA.
Lys-Asp-Pro-Val-Cys-Tyr-Leu-Lys-Lys-Ala-Phe-Leu-Leu-Val-Gln-

TAC.ATA.ATG.GAG.GAC.ACC.ATG.CGC.TTC.AGA.GAT.AAC.ACC.CCC.AAT.
Tyr-Ile-Met-Glu-Asp-Thr-Met-Arg-Phe-Arg-Asp-Asn-Thr-Pro-Asn-

GCC.ATC.GCC.ATT.GTG.CAG.CTG.CAG.GAA.CTC.TCT.TTG.AGG.CTG.AAG.
Ala-Ile-Ala-Ile-Val-Gln-Leu-Gln-Glu-Leu-Ser-Leu-Arg-Leu-Lys

AGC.TGC.TTC.ACC.AAG.GAT.TAT.GAA.GAG.CAT.GAC.AAG.GCC.TGC.GTC.
Ser-Cys-Phe-Thr-Lys-Asp-Tyr-Glu-Glu-His-Asp-Lys-Ala-Cys-Val-

CGA.ACT.TTC.TAT.GAG.ACA.CCT.CTC.CAG.TTG.CTG.GAG.AAG.GTC.AAG.
Arg-Thr-Phe-Tyr-Glu-Thr-Pro-Leu-Gln-Leu-Leu-Glu-Lys-Val-Lys-

AAT.GTC.TTT.AAT.GAA.ACA.AAG.AAT.CTC.CTT.GAC.AAG.GAC.TGG.AAT.
Asn-Val-Phe-Asn-Glu-Thr-Lys-Asn-Leu-Leu-Asp-Lys-Asp-Trp-Asn-

ATT.TTC.AGC.AAG.AAC.TGC.AAC.AAC.AGC.TTT.GCT.GAA.TGC.TCC.AGC.
Ile-Phe-Ser-Lys-Asn-Cys-Asn-Asn-Ser-Phe-Ala-Glu-Cys-Ser-Ser-

CAA.GAT.GTG.GTG.ACC.AAG.CCT.GAT.TGC.AAC.TGC.CTG.TAC.CCC.AAA.
Gln-Asp-Val-Val-Thr-Lys-Pro-Asp-Cys-Asn-Cys-Leu-Tyr-Pro-Lys-

GCC.ATC.CCT.AGC.AGT.GAC.CCG.GCC.TCT.GTC.TCC.CCT.CAT.CAG.CCC.
Ala-Ile-Pro-Ser-Ser-Asp-Pro-Ala-Ser-Val-Ser-Pro-His-Gln-Pro-

CTC.GCC.CCC.TCC.ATG.GCC.CCT.GTG.GCT.GGC.TTG.ACC.TGG.GAG.GAC.
Leu-Ala-Pro-Ser-Met-Ala-Pro-Val-Ala-Gly-Leu-Thr-Trp-Glu-Asp-

# FIG. 3-2

```
TCT.GAG.GGA.ACT.GAG.GGC.AGC.TCC.CTC.TTG.CCT.GGT.GAG.CAG.CCC.
Ser-Glu-Gly-Thr-Glu-Gly-Ser-Ser-Leu-Leu-Pro-Gly-Glu-Gln-Pro-

CTG.CAC.ACA.GTG.GAT.CCA.GGC.AGT.GCC.AAG.CAG.CGG.CCA.CCC.AGG.
Leu-His-Thr-Val-Asp-Pro-Gly-Ser-Ala-Lys-Gln-Arg-Pro-Pro-Arg-

AGC.ACC.TGC.CAG.AGC.TTT.GAG.CCG.CCA.GAG.ACC.CCA.GTT.GTC.AAG.
Ser-Thr-Cys-Gln-Ser-Phe-Glu-Pro-Pro-Glu-Thr-Pro-Val-Val-Lys-

GAC.AGC.ACC.ATC.GGT.GGC.TCA.CCA.CAG.CCT.CGC.CCC.TCT.GTC.GGG.
Asp-Ser-Thr-Ile-Gly-Gly-Ser-Pro-Gln-Pro-Arg-Pro-Ser-Val-Gly-

GCC.TTC.AAC.CCC.GGG.ATG.GAG.GAT.ATT.CTT.GAC.TCT.GCA.ATG.GGC.
Ala-Phe-Asn-Pro-Gly-Met-Glu-Asp-Ile-Leu-Asp-Ser-Ala-Met-Gly-

ACT.AAT.TGG.GTC.CCA.GAA.GAA.GCC.TCT.GGA.GAG.GCC.AGT.GAG.ATT.
Thr-Asn-Trp-Val-Pro-Glu-Glu-Ala-Ser-Gly-Glu-Ala-Ser-Glu-Ile-

CCC.GTA.CCC.CAA.GGG.ACA.GAG.CTT.TCC.CCC.TCC.AGG.CCA.GGA.GGG.
Pro-Val-Pro-Gln-Gly-Thr-Glu-Leu-Ser-Pro-Ser-Arg-Pro-Gly-Gly-

GGC.AGC.ATG.CAG.ACA.GAG.CCC.GCC.AGA.CCC.AGC.AAC.TTC.CTC.TCA.
Gly-Ser-Met-Gln-Thr-Glu-Pro-Ala-Arg-Pro-Ser-Asn-Phe-Leu-Ser-

GCA.TCT.TCT.CCA.CTC.CCT.GCA.TCA.GCA.AAG.GGC.CAA.CAG.CCG.GCA.
Ala-Ser-Ser-Pro-Leu-Pro-Ala-Ser-Ala-Lys-Gly-Gln-Gln-Pro-Ala-

GAT.GTA.ACT.GGT.ACC.GCC.TTG.CCC.AGG.GTG.GGC.CCG.TGA.
Asp-Val-Thr-Gly-Thr-Ala-Leu-Pro-Arg-Val-Gly-Pro-***-
```

29

# FIG. 4-1

```
5'  AGCCGCTCTC  CGCATCCCAG  GACAGCGGTG  CGGCCCTCGG  CCGGGGCGCC
    TCGGCGAGAG  GCGTAGGGTC  CTGTCGCCAC  GCCGGGAGCC  GGCCCCGCGG

    CACTCCGCAG  CACCCAGCGA  GCGAGCGAGC  GAGCGAGGGC  GGCCGACGCG
    GTGAGGCGTC  GTGGGTCGCT  CGCTCGCTCG  CTCGCTCCCG  CCGGCTGCGC

    CCCGGCCGGG  ACCCAGCTGC  CCGTATGACC  GCGCCGGGCG  CCGCCGGGCG
    GGGCCGGCCC  TGGGTCGACG  GGCATACTGG  CGCGGCCCGC  GGCGGCCCGC

    CTGCCCTCCC  ACGACATGGC  TGGGCTCCCT  GCTGTTGTTG  GTCTGTCTCC
    GACGGGAGGG  TGCTGTACCG  ACCCGAGGGA  CGACAACAAC  CAGACAGAGG

    TGGCGAGCAG  GAGTATCACC  GAGGAGGTGT  CGGAGTACTG  TAGCCACATG
    ACCGCTCGTC  CTCATAGTGG  CTCCTCACA   GCCTCATGAC  ATCGGTGTAC

    ATTGGGAGTG  GACACCTGCA  GTCTCTGCAG  CGGCTGATTG  ACAGTCAGAT
    TAACCCTCAC  CTGTGGACGT  CAGAGACGTC  GCCGACTAAC  TGTCAGTCTA

    GGAGACCTCG  TGCCAAATTA  CATTTGAGTT  TGTAGACCAG  GAACAGTTGA
    CCTCTGGAGC  ACGGTTTAAT  GTAAACTCAA  ACATCTGGTC  CTTGTCAACT

    AAGATCCAGT  GTGCTACCTT  AAGAAGGCAT  TTCTCCTGGT  ACAAGACATA
    TTCTAGGTCA  CACGATGGAA  TTCTTCCGTA  AAGAGGACCA  TGTTCTGTAT

    ATGGAGGACA  CCATGCGCTT  CAGAGATAAC  ACCCCCAATG  CCATCGCCAT
    TACCTCCTGT  GGTACGCGAA  GTCTCTATTG  TGGGGGTTAC  GGTAGCGGTA

    TGTGCAGCTG  CAGGAACTCT  CTTTGAGGCT  GAAGAGCTGC  TTCACCAAGG
    ACACGTCGAC  GTCCTTGAGA  GAAACTCCGA  CTTCTCGACG  AAGTGGTTCC

    ATTATGAAGA  GCATGACAAG  GCCTGCGTCC  GAACTTTCTA  TGAGACACCT
    TAATACTTCT  CGTACTGTTC  CGGACGCAGG  CTTGAAAGAT  ACTCTGTGGA

    CTCCAGTTGC  TGGAGAAGGT  CAAGAATGTC  TTTAATGAAA  CAAAGAATCT
    GAGGTCAACG  ACCTCTTCCA  GTTCTTACAG  AAATTACTTT  GTTTCTTAGA

    CCTTGACAAG  GACTGGAATA  TTTTCAGCAA  GAACTGCAAC  AACAGCTTTG
    GGAACTGTTC  CTGACCTTAT  AAAAGTCGTT  CTTGACGTTG  TTGTCGAAAC

    CTGAATGCTC  CAGCCAAGAT  GTGGTGACCA  AGCCTGATTG  CAACTGCCTG
    GACTTACGAG  GTCGGTTCTA  CACCACTGGT  TCGGACTAAC  GTTGACGGAC

    TACCCCAAAG  CCATCCCTAG  CAGTGACCCG  GCCTCTGTCT  CCCCTCATCA
    ATGGGGTTTC  GGTAGGGATC  GTCACTGGGC  CGGAGACAGA  GGGGAGTAGT
```

30

# FIG. 4-2

```
GCCCCTCGCC CCCTCCATGG CCCCTGTGGC TGGCTTGACC TGGGAGGACT
CGGGGAGCGG GGGAGGTACC GGGGACACCG ACCGAACTGG ACCCTCCTGA

CTGAGGCAAC TGAGGGCAGC TCCCTCTTGC CTGGTGAGCA GCCCCTGCAC
GACTCCCTTG ACTCCCGTCG AGGGAGAACG GACCACTCGT CGGGGACGTG

ACAGTGGATC CAGGCAGTGC CAAGCAGCGG CCACCCAGGA GCACCTGCCA
TGTCACCTAG GTCCGTCACG GTTCGTCGCC GGTGGGTCCT CGTGGACGGT

GAGCTTTGAG CCGCCAGAGA CCCCAGTTGT CAAGGACAGC ACCATCGGTG
CTCGAAACTC GGCGGTCTCT GGGGTCAACA GTTCCTGTCG TGGTAGCCAC

GCTCACCACA GCCTCGCCCC TCTGTCGGGG CCTTCAACCC CGGGATGGAG
CGAGTGGTGT CGGAGCGGGG AGACAGCCCC GGAAGTTGGG GCCCTACCTC

GATATTCTTG ACTCTGCAAT GGGCACTAAT TGGGTCCCAG AAGAAGCCTC
CTATAAGAAC TGAGACGTTA CCCGTGATTA ACCCAGGGTC TTCTTCGGAG

TGGAGAGGCC AGTGAGATTC CCGTACCCCA AGGGACAGAG CTTTCCCCCT
ACCTCTCCGG TCACTCTAAG GGCATGGGGT TCCCTGTCTC GAAAGGGGGA

CCAGGCCAGG AGGGGGCAGC ATGCAGACAG AGCCCGCCAG ACCCAGCAAC
GGTCCGGTCC TCCCCCGTCG TACGTCTGTC TCGGGCGGTC TGGGTCGTTG

TTCCTCTCAG CATCTTCTCC ACTCCCTGCA TCAGCAAAGG GCCAACAGCC
AAGGAGAGTC GTAGAAGAGG TGAGGGACGT AGTCGTTTCC CGGTTGTCGG

GGCAGATGTA ACTGGTACCG CCTTGCCCAG GGTGGGCCCG GTGAGGCCCA
CCGTCTACAT TGACCATGGC GGAACGGGTC CCACCCGGGC CACTCCGGGT

CTGGCCAGGA CTGGAATCAC ACCCCCCAGA AGACAGACCA TCCATCTGCC
GACCGGTCCT GACCTTAGTG TGGGGGGTCT TCTGTCTGGT AGGTAGACGG

CTGCTCAGAG ACCCCCCGGA GCCAGGCTCT CCCAGGATCT CATCACCGCG
GACGAGTCTC TGGGGGGCCT CGGTCCGAGA GGGTCCTAGA GTAGTGGCGC

CCCCCAGGGC CTCAGCAACC CCTCCACCCT CTCTGCTCAG CCACAGCTTT
GGGGGTCCCG GAGTCGTTGG GGAGGTGGGA GAGACGAGTC GGTGTCGAAA

CCAGAAGCCA CTCCTCGGGC AGCGTGCTGC CCCTTGGGGA GCTGGAGGGC
GGTCTTCGGT GAGGAGCCCG TCGCACGACG GGGAACCCCT CGACCTCCCG

AGGAGGAGCA CCAGGGATCG GAGGAGCCCC GCAGAGCCAG AAGGAGGACC
TCCTCCTCGT GGTCCCTAGC CTCCTCGGGG CGTCTCGGTC TTCCTCCTGG
```

31

# FIG. 4-3

```
AGCAAGTGAA GGGGCAGCCA GGCCCCTGCC CCGTTTTAAC TCCGTTCCTT
TCGTTCACTT CCCCGTCGGT CCGGGGACGG GGCAAAATTG AGGCAAGGAA

TGACTGACAC AGGCCATGAG AGGCAGTCCG AGGGATCCTC CAGCCCGCAG
ACTGACTGTG TCCGGTACTC TCCGTCAGGC TCCCTAGGAG GTCGGGCGTC

CTCCAGGAGT CTGTCTTCCA CCTGCTGGTG CCCAGTGTCA TCCTGGTCTT
GAGGTCCTCA GACAGAAGGT GGACGACCAC GGGTCACAGT AGGACCAGAA

GCTGGCCGTC GGAGGCCTCT TGTTCTACAG GTGGAGGCGG CGGAGCCATC
CGACCGGCAG CCTCCGGAGA ACAAGATGTC CACCTCCGCC GCCTCGGTAG

AAGAGCCTCA GAGAGCGGAT TCTCCCTTGG AGCAACCAGA GGGCAGCCCC
TTCTCGGAGT CTCTCGCCTA AGAGGGAACC TCGTTGGTCT CCCGTCGGGG

CTCACTCAGG ATGACAGACA GGTGGAACTG CCAGTGTAGA GGGAATTCTA
GAGTGAGTCC TACTGTCTGT CCACCTTGAC GGTCACATCT CCCTTAAGAT

AGACCCCTCA CCATCCTGGA CACTCTCGTT TGTCAATGTC CCTCTGAAAA
TCTGGGGAGT GGTAGGACCT GTGAGACCAA ACAGTTACAG GGAGACTTTT

TGTGACGCCC AGCCCCGGAC ACAGTACTCC AGATGTTGTC TGACCAGCTC
ACACTGCGGG TCGGGGCCTG TGTCATGAGG TCTACAACAG ACTGGTCGAG

AGAGAGAGTA CAGTGGGACT GTTACCTTCC TTGATATGGA CAGTATTCTT
TCTCTCTCAT GTCACCCTGA CAATGGAAGG AACTATACCT GTCATAAGAA

CTATTTGTGC AGATTAAGAT TGCATTAGTT TTTTTCTTAA CAACTGCATC
GATAAACACG TCTAATTCTA ACGTAATCAA AAAAAGAATT GTTGACGTAG

ATACTGTTGT CATATGTTGA GCCTGTGGTC TATAAAACCC CTAGTTCCAT
TATGACAACA GTATACAACT CGGACACCAG ATATTTGGG GATCAAGGTA

TTCCCATAAA CTTCTGTCAA GCCAGACCAT CTCTACCCTG TACTTGGACA
AAGGGTATTT GAAGACAGTT CGGTCTGGTA GAGATGGGAC ATGAACCTGT

ACTTAACTTT TTTAACCAAA GTGCAGTTTA TGTTCACCTT TGTTAAAGCC
TGAATTGAAA AAATTGGTTT CACGTCAAAT ACAAGTGGAA ACAATTTCGG

ACCTTGTGGT TTCTGCCCAT CACCTGAACC TACTGAAGTT GTGTGAAATC
TGGAACACCA AAGACGGGTA GTGGACTTGG ATGACTTCAA CACACTTTAG

CTAATTCTGT CATCTCCGTA GCCCTCCCAG TTGTGCCTCC TGCACATTGA
GATTAAGACA GTAGAGGCAT CGGGAGGGTC AACACGGAGG ACGTGTAACT
```

32

# FIG. 4-4

```
TGAGTGCCTG CTGTTGTCTT TGCCCATGTT GTTGATGTAG CTGTGACCCT
ACTCACGGAC GACAACAGAA ACGGGTACAA CAACTACATC GACACTGGGA

ATTGTTCCTC ACCCCTGCCC CCCGCCAACC CCAGCTGGCC CACCTCTTCC
TAACAAGGAG TGGGGACGGG GGGCGGTTGG GGTCGACCGG GTGGAGAAGG

CCCTCCCACC CAAGCCCACA GCCAGCCCAT CAGGAAGCCT TCCTGGCTTC
GGGAGGGTGG GTTCGGGTGT CGGTCGGGTA GTCCTTCGGA AGGACCGAAG

TCCACAACCT TCTGACTGTC TTTTCAGTCA TGCCCCCTGC TCTTTTGTAT
AGGTGTTGGA AGACTGACAG AAAAGTCAGT ACGGGGGACG AGAAAACATA

TTGGCTAATA GTATATCAAT TTGCACTTAA AAAAAAAAAA AAAAAAAAAA 3'
AACCGATTAT CATATAGTTA AACGTGAATT TTTTTTTTTT TTTTTTTTTT
```

33

# FIG. 5-1

```
ATG.ACC.GCG.CCG.GGC.GCC.GCC.GGG.CGC.TGC.CCT.CCC.ACG.ACA.TGG.
Met-Thr-Ala-Pro-Gly-Ala-Ala-Gly-Arg-Cys-Pro-Pro-Thr-Thr-Trp-

CTG.GGC.TCC.CTG.CTG.TTG.TTG.GTC.TGT.CTC.CTG.GCG.AGC.AGG.AGT.
Leu-Gly-Ser-Leu-Leu-Leu-Leu-Val-Cys-Leu-Leu-Ala-Ser-Arg-Ser-

ATC.ACC.GAG.GAG.GTG.TCG.GAG.TAC.TGT.AGC.CAC.ATG.ATT.GGG.AGT.
Ile-Thr-Glu-Glu-Val-Ser-Glu-Tyr-Cys-Ser-His-Met-Ile-Gly-Ser-

GGA.CAC.CTG.CAG.TCT.CTG.CAG.CGG.CTG.ATT.GAC.AGT.CAG.ATG.GAG.
Gly-His-Leu-Gln-Ser-Leu-Gln-Arg-Leu-Ile-Asp-Ser-Gln-Met-Glu-

ACC.TCG.TGC.CAA.ATT.ACA.TTT.GAG.TTT.GTA.GAC.CAG.GAA.CAG.TTG.
Thr-Ser-Cys-Gln-Ile-Thr-Phe-Glu-Phe-Val-Asp-Gln-Glu-Gln-Leu-

AAA.GAT.CCA.GTG.TGC.TAC.CTT.AAG.AAG.GCA.TTT.CTC.CTG.GTA.CAA.
Lys-Asp-Pro-Val-Cys-Tyr-Leu-Lys-Lys-Ala-Phe-Leu-Leu-Val-Gln-

GAC.ATA.ATG.GAG.GAC.ACC.ATG.CGC.TTC.AGA.GAT.AAC.ACC.CCC.AAT.
Asp-Ile-Met-Glu-Asp-Thr-Met-Arg-Phe-Arg-Asp-Asn-Thr-Pro-Asn-

GCC.ATC.GCC.ATT.GTG.CAG.CTG.CAG.GAA.CTC.TCT.TTG.AGG.CTG.AAG.
Ala-Ile-Ala-Ile-Val-Gln-Leu-Gln-Glu-Leu-Ser-Leu-Arg-Leu-Lys

AGC.TGC.TTC.ACC.AAG.GAT.TAT.GAA.GAG.CAT.GAC.AAG.GCC.TGC.GTC.
Ser-Cys-Phe-Thr-Lys-Asp-Tyr-Glu-Glu-His-Asp-Lys-Ala-Cys-Val-

CGA.ACT.TTC.TAT.GAG.ACA.CCT.CTC.CAG.TTG.CTG.GAG.AAG.GTC.AAG.
Arg-Thr-Phe-Tyr-Glu-Thr-Pro-Leu-Gln-Leu-Leu-Glu-Lys-Val-Lys-

AAT.GTC.TTT.AAT.GAA.ACA.AAG.AAT.CTC.CTT.GAC.AAG.GAC.TGG.AAT.
Asn-Val-Phe-Asn-Glu-Thr-Lys-Asn-Leu-Leu-Asp-Lys-Asp-Trp-Asn-

ATT.TTC.AGC.AAG.AAC.TGC.AAC.AAC.AGC.TTT.GCT.GAA.TGC.TCC.AGC.
Ile-Phe-Ser-Lys-Asn-Cys-Asn-Asn-Ser-Phe-Ala-Glu-Cys-Ser-Ser-

CAA.GAT.GTG.GTG.ACC.AAG.CCT.GAT.TGC.AAC.TGC.CTG.TAC.CCC.AAA.
Gln-Asp-Val-Val-Thr-Lys-Pro-Asp-Cys-Asn-Cys-Leu-Tyr-Pro-Lys-

GCC.ATC.CCT.AGC.AGT.GAC.CCG.GCC.TCT.GTC.TCC.CCT.CAT.CAG.CCC.
Ala-Ile-Pro-Ser-Ser-Asp-Pro-Ala-Ser-Val-Ser-Pro-His-Gln-Pro-

CTC.GCC.CCC.TCC.ATG.GCC.CCT.GTG.GCT.GGC.TTG.ACC.TGG.GAG.GAC.
Leu-Ala-Pro-Ser-Met-Ala-Pro-Val-Ala-Gly-Leu-Thr-Trp-Glu-Asp-
```

34

# FIG. 5-2

TCT.GAG.GGA.ACT.GAG.GGC.AGC.TCC.CTC.TTG.CCT.GGT.GAG.CAG.CCC.
Ser-Glu-Gly-Thr-Glu-Gly-Ser-Ser-Leu-Leu-Pro-Gly-Glu-Gln-Pro-

CTG.CAC.ACA.GTG.GAT.CCA.GGC.AGT.GCC.AAG.CAG.CGG.CCA.CCC.AGG.
Leu-His-Thr-Val-Asp-Pro-Gly-Ser-Ala-Lys-Gln-Arg-Pro-Pro-Arg-

AGC.ACC.TGC.CAG.AGC.TTT.GAG.CCG.CCA.GAG.ACC.CCA.GTT.GTC.AAG.
Ser-Thr-Cys-Gln-Ser-Phe-Glu-Pro-Pro-Glu-Thr-Pro-Val-Val-Lys-

GAC.AGC.ACC.ATC.GGT.GGC.TCA.CCA.CAG.CCT.CGC.CCC.TCT.GTC.GGG.
Asp-Ser-Thr-Ile-Gly-Gly-Ser-Pro-Gln-Pro-Arg-Pro-Ser-Val-Gly-

GCC.TTC.AAC.CCC.GGG.ATG.GAG.GAT.ATT.CTT.GAC.TCT.GCA.ATG.GGC.
Ala-Phe-Asn-Pro-Gly-Met-Glu-Asp-Ile-Leu-Asp-Ser-Ala-Met-Gly-

ACT.AAT.TGG.GTC.CCA.GAA.GAA.GCC.TCT.GGA.GAG.GCC.AGT.GAG.ATT.
Thr-Asn-Trp-Val-Pro-Glu-Glu-Ala-Ser-Gly-Glu-Ala-Ser-Glu-Ile-

CCC.GTA.CCC.CAA.GGG.ACA.GAG.CTT.TCC.CCC.TCC.AGG.CCA.GGA.GGG.
Pro-Val-Pro-Gln-Gly-Thr-Glu-Leu-Ser-Pro-Ser-Arg-Pro-Gly-Gly-

GGC.AGC.ATG.CAG.ACA.GAG.CCC.GCC.AGA.CCC.AGC.AAC.TTC.CTC.TCA.
Gly-Ser-Met-Gln-Thr-Glu-Pro-Ala-Arg-Pro-Ser-Asn-Phe-Leu-Ser-

GCA.TCT.TCT.CCA.CTC.CCT.GCA.TCA.GCA.AAG.GGC.CAA.CAG.CCG.GCA.
Ala-Ser-Ser-Pro-Leu-Pro-Ala-Ser-Ala-Lys-Gly-Gln-Gln-Pro-Ala-

GAT.GTA.ACT.GGT.ACC.GCC.TTG.CCC.AGG.GTG.GGC.CCC.GTG.AGG.CCC.
Asp-Val-Thr-Gly-Thr-Ala-Leu-Pro-Arg-Val-Gly-Pro-Val-Arg-Pro-

ACT.GGC.CAG.GAC.TGG.AAT.CAC.ACC.CCC.CAG.AAG.ACA.GAC.CAT.CCA.
Thr-Gly-Gln-Asp-Trp-Asn-His-Thr-Pro-Gln-Lys-Thr-Asp-His-Pro-

TCT.GCC.CTG.CTC.AGA.GAC.CCC.CCG.GAG.CCA.GGC.TCT.CCC.AGG.ATC.
Ser-Ala-Leu-Leu-Arg-Asp-Pro-Pro-Glu-Pro-Gly-Ser-Pro-Arg-Ile-

TCA.TCA.CCG.CGC.CCC.CAG.GGC.CTC.AGC.AAC.CCC.TCC.ACC.CTC.TCT.
Ser-Ser-Pro-Arg-Pro-Gln-Gly-Leu-Ser-Asn-Pro-Ser-Thr-Leu-Ser-

GCT.CAG.CCA.CAG.CTT.TCC.AGA.AGC.CAC.TCC.TCG.GGC.AGC.GTG.CTG.
Ala-Gln-Pro-Gln-Leu-Ser-Arg-Ser-His-Ser-Ser-Gly-Ser-Val-Leu-

CCC.CTT.GGG.GAG.CTG.GAG.GGC.AGG.AGG.AGC.ACC.AGG.GAT.CGG.AGG.
Pro-Leu-Gly-Glu-Leu-Glu-Gly-Arg-Arg-Ser-Thr-Arg-Asp-Arg-Arg-

# FIG. 5-3

```
AGC.CCC.GCA.GAG.CCA.GAA.GGA.GGA.CCA.GCA.AGT.GAA.GGG.GCA.GCC.
Ser-Pro-Ala-Glu-Pro-Glu-Gly-Gly-Pro-Ala-Ser-Glu-Gly-Ala-Ala-

AGG.CCC.CTG.CCC.CGT.TTT.AAC.TCC.GTT.CCT.TTG.ACT.GAC.ACA.GGC.
Arg-Pro-Leu-Pro-Arg-Phe-Asn-Ser-Val-Pro-Leu-Thr-Asp-Thr-Gly-

CAT.GAG.AGG.CAG.TCC.GAG.GGA.TCC.TCC.AGC.CCG.CAG.CTC.CAG.GAG.
His-Glu-Arg-Gln-Ser-Glu-Gly-Ser-Ser-Ser-Pro-Gln-Leu-Gln-Glu-

TCT.GTC.TTC.CAC.CTG.CTG.GTG.CCC.AGT.GTC.ATC.CTG.GTC.TTG.CTG.
Ser-Val-Phe-His-Leu-Leu-Val-Pro-Ser-Val-Ile-Leu-Val-Leu-Leu-

GCC.GTC.GGA.GGC.CTC.TTG.TTC.TAC.AGG.TGG.AGG.CGG.CGG.AGC.CAT.
Ala-Val-Gly-Gly-Leu-Leu-Phe-Tyr-Arg-Trp-Arg-Arg-Arg-Ser-His-

CAA.GAG.CCT.CAG.AGA.GCG.GAT.TCT.CCC.TTG.GAG.CAA.CCA.GAG.GGC.
Gln-Glu-Pro-Gln-Arg-Ala-Asp-Ser-Pro-Leu-Glu-Gln-Pro-Glu-Gly-

AGC.CCC.CTC.ACT.CAG.GAT.GAC.AGA.CAG.GTG.GAA.CTG.CCA.GTG.TAG.
Ser-Pro-Leu-Thr-Gln-Asp-Asp-Arg-Gln-Val-Glu-Leu-Pro-Val-***-
```

36

# FIG. 6

FIG. 7

EP 0 547 026 A1

FIG. 8

early

Hind III sv40
ori
EcoRI

pcDM·CSF 11

BstE II

polyA
EcoRI

EcoRI

EcoRI, BstEII
Isolate 670 bp
Fragment

Synthetic Linker

```
               BamHI   EcoRI
                 ▼       ▼
GTGACC TGATAA GGATCCG
       GACTATT CCTAGGCTTAA
   ▲
BstEII
```

```
 1      35                                178   185           BamHI   EcoRI
Met    Val                                Cys   Thr  STOP       ▼       ▼
AATTC---ATG---GTG------------------------- TGC---ACC TGATAA GGATCCG
 G---TAC---CAC------------------------- ACG---TGGACTATT CCTAGGCTTAA
 ▲
EcoRI
```

EcoRI ———— PCDE

pcDM·CSFII 185

EP 0 547 026 A1

FIG. 9-1

# FIG. 9-2

OmpA Signal Peptide

- - - |ATG - - - GTAGCACAGGCC|GGAATTCCAGCC - - - 

Met - - -Val Ala Gln Ala

M-CSF

|ATGACC - - - GAGGTGTCGGAG - - -

Met Thr - - -Glu val Ser Glu- - -

Site - Specific Mutagenesis

OmpA Signal Peptide     M-CSF

- - -|ATG - - - GTAGCACAGGCC|GTGTCGGAG - - -

Met - - -Val Ala Gln Ala|Val Ser Glu- - -

# FIG. 9-3

OmpA Signal Peptide     M-CSF

```
---|ATG---GTAGCACAGGCC|GTGTCGGAG---
   |Met---ValAlaGlnAla|ValSerGru
```

M13IG

pUC118-OmpA
-MCSF11-NV151

xbaI

BamHI

xbaI
BamHI

xbaI

BamHI

pIN-Ⅲ(Ipp$^p$-5)-A3

Ipp$^p$-5
lac$^{po}$

lacI

xbaI

BamHI

pIN-Ⅲ(Ipp$^p$-5)-OmpA
-MCSF11-NV151

Ipp$^p$-5
lac$^{po}$

lacI

EP 0 547 026 A1

# FIG. 10

Met-Lys-Lys-Thr-Ala-Ile-Ala-Ile-Ala-Val-Ala-Leu-Ala-Gly-Phe-

Ala-Thr-Val-Ala-Gln-Ala↓-Val-Ser-Glu-Tyr-Cys-Ser-His-Met-Ile-

Gly-Ser-Gly-His-Leu-Gln-Ser-Leu-Gln-Arg-Leu-Ile-Asp-Ser-Gln-

Met-Glu-Thr-Ser-Cys-Gln-Ile-Thr-Phe-Glu-Phe-Val-Asp-Gln-Glu-

Gln-Leu-Lys-Asp-Pro-Val-Cys-Tyr-Leu-Lys-Lys-Ala-Phe-Leu-Leu-

Val-Gln-Asp-Ile-Met-Glu-Asp-Thr-Met-Arg-Phe-Arg-Asp-Asn-Thr-

Pro-Asn-Ala-Ile-Ala-Ile-Val-Gln-Leu-Gln-Glu-Leu-Ser-Leu-Arg-

Leu-Lys-Ser-Cys-Phe-Thr-Lys-Asp-Tyr-Glu-Glu-His-Asp-Lys-Ala-

Cys-Val-Arg-Thr-Phe-Tyr-Glu-Thr-Pro-Leu-Gln-Leu-Leu-Glu-Lys-

Val-Lys-Asn-Val-Phe-Asn-Glu-Thr-Lys-Asn-Leu-Leu-Asp-Lys-Asp-

Trp-Asn-Ile-Phe-Ser-Lys-Asn-Cys-Asn-Asn-Ser-Phe-Ala-Glu-Cys-

Ser-Ser-Gln-Asp-Val-Val-Thr

# FIG. 11

Met-Lys-Lys-Thr-Ala-Ile-Ala-Ile-Ala-Val-Ala-Leu-Ala-Gly-Phe-

Ala-Thr-Val-Ala-Gln-Ala↓Val-Ser-Glu-Tyr-Cys-Ser-His-Met-Ile-

Gly-Ser-Gly-His-Leu-Gln-Ser-Leu-Gln-Arg-Leu-Ile-Asp-Ser-Gln-

Met-Glu-Thr-Ser-Cys-Gln-Ile-Thr-Phe-Glu-Phe-Val-Asp-Gln-Glu-

Gln-Leu-Lys-Asp-Pro-Val-Cys-Tyr-Leu-Lys-Lys-Ala-Phe-Leu-Leu-

Val-Gln-Asp-Ile-Met-Glu-Asp-Thr-Met-Arg-Phe-Arg-Asp-Asn-Thr-

Pro-Asn-Ala-Ile-Ala-Ile-Val-Gln-Leu-Gln-Glu-Leu-Ser-Leu-Arg-

Leu-Lys-Ser-Cys-Phe-Thr-Lys-Asp-Tyr-Glu-Glu-His-Asp-Lys-Ala-

Cys-Val-Arg-Thr-Phe-Tyr-Glu-Thr-Pro-Leu-Gln-Leu-Leu-Glu-Lys-

Val-Lys-Asn-Val-Phe-Asn-Glu-Thr-Lys-Asn-Leu-Leu-Asp-Lys-Asp-

Trp-Asn-Ile-Phe-Ser-Lys-Asn-Cys-Asn-Asn-Ser-Phe-Ala-Glu-

# FIG. 12-1

# FIG. 12-2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4 ) |
|---|---|---|---|
| A | WO-A-8 604 607 (CETUS CORPORATION) <br> * the whole document * <br> --- | 1-8 | C12N15/27 <br> C12P21/02 <br> C07K13/00 |
| P,A | Section Ch, Week 8735, <br> Derwent Publications Ltd., London, GB; <br> Class B04, AN 87-247006 <br> & JP-A-62 169 799 (OTSUKA PHARM KK) 25 July 1987 <br> * abstract * <br> ----- | 1-8 | C12N1/21 |

TECHNICAL FIELDS SEARCHED (Int. Cl.4 )

C07K
C12N
C12P

The present search report has been drawn up for all claims

| Place of search | Date of completion of the search | Examiner |
|---|---|---|
| THE HAGUE | 04 MARCH 1993 | LE CORNEC N.D.R. |